# EUROPÄISCHE PATENTANMELDUNG

(11) **EP 2 308 893 A1**
(43) Veröffentlichungstag der Anmeldung: **13.04.2011**
(21) Anmeldenummer: 10185302.6
(22) Anmeldetag: 05.01.2001
(51) Int. Cl.: C07K 14/705, C07K 14/395

(54) **Kalium-Kanal-Mutanten der Hefe Saccharomyces cerevisiae und deren Verwendung für das Screening von eukaryotischen Kaliumkanälen**

(30) Priorität: 11.01.2000 DE 10000651
(62) Teilanmeldung aus: 01942366.4
(71) Anmelder: Sanofi-Aventis Deutschland GmbH, 65929 Frankfurt (DE)
(72) Erfinder: Leberer, Ekkehard, 65926 Frankfurt am Main (DE); Leeuw, Thomas, 65926 Frankfurt am Main (DE); Ritscher, Allegra, 65926 Frankfurt am Main (DE)

(57) **Zusammenfassung**

Die Erfindung betrifft Verfahren zur Identifizierung von Inhibitoren und Aktivatoren eukaryotischer Kaliumkanäle, wobei eine mutierte S. cerevisiae Zelle verwendet wird, deren endogene Kaliumkanäle TRK1, TRK2 und TOK1 nicht funktionell exprimiert werden, die aber einen zu untersuchenden eukaryotischen Kaliumkanal heterolog exprimert. Desweiteren sind mutierte S. cerevisiae Zellen, die TRK1, TRK2 und TOK1 nicht exprimieren, Gegenstand der Erfindung sowie die Herstellung und Verwendung dieser mutierten S. cerevisiae Zellen.

## Beschreibung

Die Erfindung betrifft Verfahren zur Identifizierung von Inhibitoren und Aktivatoren eukaryotischer Kaliumkanäle, wobei eine mutierte S. cerevisiae Zelle verwendet wird, deren endogene Kaliumkanäle TRK1, TRK2 und TOK1 nicht funktionell exprimiert werden, die aber einen zu untersuchenden eukaryotischen Kaliumkanal heterolog exprimiert. Desweiteren sind mutierte S. cerevisiae Zellen, die TRK1, TRK2 und TOK1 nicht exprimieren, Gegenstand der Erfindung sowie die Herstellung und Verwendung dieser mutierten S. cerevisiae Zellen.

Jede Zelle ist von einer Plasmamembran umschlossen, die eine Dicke von etwa 6 - 8 nm besitzt. Diese Membran legt die Ausmaße der Zelle fest und trennt den Inhalt der Zelle von ihrer Umgebung. Alle biologischen Membranen bestehen aus einer zusammenhängenden Doppelschicht von Lipidmolekülen, in die verschiedene Membranproteine eingelagert sind. Während die Lipid-Doppelschicht die Grundstruktur biologischer Membranen bestimmt, sind die Proteine für die meisten ihrer Funktionen verantwortlich. Wegen ihres hydrophoben Innern wirkt die Lipid-Doppelschicht für die meisten polaren Moleküle als eine undurchlässige Barriere. Erst Membranproteine wie Rezeptoren, lonenkanäle und Transporter erlauben einen kontrollierten lonenfluß und den Transport von polaren Molekülen (Alberts *et al.,* 1995). Damit tragen Proteine zu unterschiedlichen lonenkonzentrationen im Innern und in der Umgebung der Zelle bei und steuern den Eintritt von Nährstoffen und den Austritt von Abbaustoffen.
Die meisten Membranproteine durchspannen die Plasmamembran mehrmals, so auch die lonenkanäle, die damit zur Gruppe der integralen Membranproteine gezählt werden. Diese Proteine besitzen sowohl hydrophobe Bereiche, die die Lipid-Doppelschicht durchspannen, als auch hydrophile Abschnitte, die auf beiden Seiten der Membran dem wäßrigen Milieu ausgesetzt sind. lonenkanäle kommen in allen Zellen vor und sind in Nervenzellen für die Generierung von Aktionspotentialen verantwortlich (Alberts *et al.,* 1995). lonenkanäle können aufgrund ihrer unterschiedlichen lonenselektivität und anhand ihrer unterschiedlichen Öffnungs- und Schließmechanismen unterschieden werden.
Kaliumkanäle sind ubiquitäre Membranproteine, die sowohl in erregbaren, als auch in nicht erregbaren Zellen vorkommen (for review see (Jan, L. Y. et al., 1997). Offene Kaliumkanäle bewegen das Membranpotential näher zum Kalium-Gleichgewichtspotential und damit weg vom Schwellenpotential zur Auslösung eines Aktionspotentials. Kaliumkanäle festigen also das Ruhemembranpotential, repolarisieren die Zelle und bestimmen damit die Länge und die Frequenz von Aktionspotentialen (Sanguinetti, M. C. et al., 1997; Wilde, A. A. et al., 1997; Wang, Q. et al., 1998). Aufgrund dieser Funktionen stellen Kaliumkanäle auch die molekulare Ursache für die Entstehung vieler pathologischer Situationen dar, und sind somit ein interessantes Ziel für die Entwicklung therapeutischer Agens.

Die Hefe *Saccharomyces cerevisiae* (nachfolgend S. cerevisiae) besitzt drei Kaliumkanäle, TRK1, TRK2 und TOK1. Der Kaliumkanal TRK1 (YJL129c) gehört zur Familie der "Major Facilitator" Kaliumpermeasen und ist als hochaffiner Kaliumtransporter verantwortlich für den Einstrom von Kaliumionen aus dem Medium in die Zelle (Gaber, R. F. et al., 1988; Ko, C. H. et al., 1990; Ko, C. H. et al., 1991). Die Deletionsmutante Δ*trk1* ist auf mindestens 10 mM K⁺ überlebensfähig und stark polarisiert (Gaber, R. F. et al., 1988; Madrid, R. et al., 1998). Auf 1 mM K⁺ überlebt ein Δ*trk1* Stamm nicht (Gaber, R. F. et al., 1988).
Der Kaliumkanal TRK2 (YKR050w) gehört ebenfalls zur Familie der "Major Facilitator" - Kaliumpermeasen und ist als niederaffiner Kaliumtransporter verantwortlich für den Einstrom von Kaliumionen aus dem Medium in die Zelle (Ko, C. H. et al., 1990; Ko, C. H. et al., 1991; Madrid, R. et al., 1998). Der Phänotyp der Δ*trk2* Deletionsmutante ist weniger stark ausgeprägt als für die Δ*trk1*-Mutante. Ein Δ*trk2* Stamm überlebt auch auf 1 mM K⁺ (Ko, C. H. et al., 1990; Madrid, R. et al., 1998).
Der Kaliumkanal TOK1 (auch unter dem Namen DVK1 oder YORK bekannt) ist verantwortlich für den Einstrom von Kaliumionen aus dem Medium in die Zelle (Ketchum, K. A. et al., 1995; Fairman, C. et al., 1999). Die Richtung des lonenströme ist jedoch reversibel, und kann daher je nach Kulturbedingungen auch in die andere Richtung leiten (Fairman, C. et al., 1999).
Die Deletionsmutante Δ*trk1* Δ*trk2* wurde schon wiederholt beschrieben (Ko, C. H. et al., 1990; Ko, C. H. et al., 1991; Madrid, R. et al., 1998; Fairman, C. et al., 1999). Diese Mutante wurde in der Vergangenheit auch dazu benutzt, K⁺ -Kanäle höherer Eukaryoten durch Komplementation des Phänotyps zu identifizieren und zu beschreiben. Bislang berichtet wurde die Komplementation durch die *inward rectifier-*Kanäle KAT1 cDNA (*Arabidopsis thaliana*)*,* HKT1 cDNA (*Triticum aestivium*)*,* IRK1 (*Mus musculus*) und HKT1 K⁺/Na⁺-Transporter (*Triticum aestivium*) (Tang, W. et al., 1995; Smith, F. W. et al., 1995; Goldstein, S. A. et al., 1996; Nakamura, R. L. et al., 1997). Zudem wurde beschrieben, daß Überexpression von TOK1 und dessen Homolog ORK1 aus Drosophila melanogaster, in der Hefezelle den Wachstumsdefekt der Δ*trk1* Δ*trk2* -Mutante komplementieren kann (Fairman, C. et al., 1999).

Die Untersuchung einer Vielzahl von eukaryotischen Kaliumkanälen und Identifizierung von Substanzen, die die Aktivität dieser Kaliumkanäle modifzieren können gestaltet sich jedoch schwierig, da z. B. die humanen Kanäle HERG1 oder Kv1.5 den lethalen Phänotyp von Δ*trk1* Δ*trk2* auf 5 mM KCl nicht komplementieren können. Somit ist kein Screening möglich.

Die Erfindung betrifft ein Verfahren zur Identifizierung von Inhibitoren eines eukaryotischen Kaliumkanals, wobei
a) eine mutierte S. cerevisiae Zelle verwendet wird, die die drei endogenen Kaliumkanäle TRK1, TRK2 und TOK1 nicht exprimiert;
b) in dieser mutierten S. cerevisiae Zelle ein eukaryotischer Kaliumkanal heterolog exprimiert wird;
c) die mutierte S. cerevisiae Zelle mit einer zu untersuchenden Substanz inkubiert wird;
   und
d) der Effekt der zu untersuchenden Substanz auf den eukaryotischen Kaliumkanal bestimmt wird.

In der in dem Verfahren verwendeten mutierte S. cerevisiae Zelle sind die Gene TRK1, TRK2 und TOK1 (SEQ ID NO. 1, SEQ ID NO. 2 und SEQ ID NO. 3)
ausgeschaltet (Δtrk1, Δtrk2, Δtok1), vorzugsweise durch knock out, wobei vorzugsweise große Teile der Gene deletiert werden.

Der in dem Verfahren verwendete eukaryotische Kaliumkanal ist der Kaliumkanal, der untersucht werden soll - der Kanal, für den Inhibitoren oder Aktivatoren identifiziert werden sollen.

Der eukaryotische Kaliumkanal ist beispielsweise ein humaner HERG1, humaner Kv1.5, humaner ROMK2 oder gpIRK1 (Meerschweinchen) Kanal. Der eukaryotische Kaliumkanal hat vorzugsweise die natürliche Sequenz des betreffenden Kaliumkanals, beispielsweise kodiert durch eine der Sequenzen SEQ ID NO. 4, SEQ ID. NO. 5, SEQ ID NO. 7 (ROMK2) oder SEQ ID NO. 6. Die natürliche Sequenz des Kaliumkanals kann aber auch modifiziert, z.B. mutiert sein.
Vorzugsweise wird die Nukleotidsequenz, die für den eukaryotischen Kaliumkanal kodiert, in ein Hefe Expressionsplasmid, beispielsweise p423 GPD3 oder einen Vektor beispielsweise der pRS 42x- oder pRS 32x-Serie integriert und das rekombinante Expressionsplasmid in die mutierte S. cerevisiae Zelle eingebracht.

Mit dem Verfahren sollen Substanzen identifiziert werden, die auf den eukaryotischen Kaliumkanal einen Effekt haben. Diese Substanzen inhibieren das Wachstum der mutierten S. cerevisiae Zelle. Eine zu untersuchende Substanz, die den heterolog exprimierten eukaryotischen Kaliumkanal inhibiert, bewirkt, daß sich die mutierte S. cerevisiae Zelle - da sie keine endogenen Kaliumkanäle exprimiert - schlechter bzw. langsamer teilt und vermehrt bzw. in einer besonderen Ausführungsform der Erfindung abstirbt.

Der Effekt der zu untersuchenden Substanz kann beispielsweise direkt über Messung der optischen Dichte bei 600 nm oder mit Hilfe eines konstitutiv in der mutierten S. cerevisiae Zelle exprimierten Wachstumsreporters bestimmt werden. Vorzugsweise kodiert der konstitutive exprimierte Wachstumsreporter für ein Protein, das entweder selbst Fluoreszenz oder Lumineszenz zeigt oder das an einer Reaktion beteiligt ist, die ein Fluoreszenz oder Lumineszenz-Signal liefert. Die für den Wachstumsreporter kodierende Sequenz liegt vorzugsweise in einem Vektor vor. Als Wachstumsreporter sind beispielsweise das LacZ Gen für β-Galaktosidase oder die saure Phosphatase PH03 geeignet, die unter der Kontrolle eines konstitutiven Hefe Promotors exprimiert werden. Aus der meßbaren Fluoreszenz oder Lumineszenz kann auf die Zellzahl der mutierten S. cerevisiae Zellen geschlossen werden. Wird keine bzw. weniger Fluoreszenz bzw. Lumineszenz gemessen, dann sind in der betreffenden Probe weniger mutierte S. cerevisiae Zellen vorhanden. Sind weniger mutierte S. cerevisiae Zellen vorhanden, dann hat die zu untersuchende Substanz einen inhibierenden Effekt auf den eukaryotischen Kaliumkanal.

Die beschriebenen Verfahren lassen sich besonders gut automatisieren und für eine Vielzahl von zu untersuchenden Substanzen parallel durchführen. In besonderen Ausführungsformen der Erfindung werden zwei oder mehrere Verfahren vergleichend durchgeführt, wobei zwei oder mehr mutierte S. cerevisiae Zellen vergleichend analysiert werden. Diese mutierten S. cerevisiae Zellen werden vorzugsweise mit der gleich Menge an zu untersuchender Substanz inkubiert, exprimieren aber den betreffenden eukaryotischen Kaliumkanal in unterschiedlichem Maße. In einer anderen besonderen Ausführungsform der Erfindung werden mutierte S. cerevisiae Zellen vergleichend analysiert, die den betreffenden eukaryotischen Kaliumkanal in gleichem Maße exprimieren, aber mit unterschiedlichen Mengen an zu untersuchender Substanz inkubiert werden.

Gegenstand der Erfindung ist auch eine mutierte S. cerevisiae Zelle, in der die endogenen Kaliumkanäle TRK1, TRK2 und TOK1 nicht exprimiert werden. Eine weitere Ausführungsform betrifft eine mutierte S. cerevisiae Zelle, in der die Gene TRK1, TRK2 und TOK1 ausgeschaltet sind; vorzugsweise sind diese Gene durch knock out ganz oder teilweise entfernt oder wurden mutiert. Eine weitere Ausführungsform betrifft eine mutierte S. cerevisiae Zelle, die nach dem Budapester Vertrag über die internationale Anerkennung der Hinterlegung von Mikroorganismen für die Zwecke von Patentverfahren bei der Deutschen Sammlung von Mikroorganismen und Zellkulturen GmbH (Mascheroder Weg 1 b, D-38124 Braunschweig) unter DSM 13197 hinterlegt ist. Eine besondere Ausführungsform der Erfindung betrifft eine mutierte S. cerevisiae Zelle, die einen eukaryotischen Kaliumkanal heterolog exprimiert, wobei der eukaryotische Kaliumkanal vorzugsweise ein humaner Kaliumkanal ist, beispielsweise ein HERG1, Kv1.5 oder gpIRK1 oder ein humaner Kv 4.3 [Genbank Zugangsnummer AF 187963], TASK [Genbank Zugangsnummer AF 006823] oder ROMK2 [Genbank Zugangsnummer U 12542] ist und wobei der Kaliumkanal die natürliche Sequenz hat oder mutiert sein kann.

Die Erfindung betrifft auch ein Verfahren zur Herstellung einer mutierten S. cerevisiae Zelle, die die Kaliumkanäle TRK, TRK2 und TOK1 nicht exprimiert, wobei die Gene TRK1, TRK2 und TOK1 durch knock out zerstört bzw. deletiert werden.
Die mutierte S. cerevisiae Zelle kann beispielsweise in Verfahren zur Identifizierung von Substanzen, die die Aktivität des eukaryotischen Kaliumkanals inhibieren oder aktivieren, verwendet werden oder Bestandteil eines Test-Kits sein, der z.B. zur Bestimmung von toxischen Substanzen verwendet werden kann.

Die Erfindung betrifft auch ein Verfahren zur Identifizierung von Aktivatoren eines eukaryotischen Kaliumkanals, wobei
a) eine mutierte S. cerevisiae Zelle verwendet wird, die die drei endogenen Kaliumkanäle TRK1, TRK2 und TOK1 nicht exprimiert;
b) in dieser mutierten S. cerevisiae Zelle ein eukaryotischer Kaliumkanal heterolog exprimiert wird;
c) die mutierte S. cerevisiae Zelle mit einer zu untersuchenden Substanz inkubiert wird;
   und
d) der Effekt der zu untersuchenden Substanz auf den eukaryotischen Kaliumkanal bestimmt wird.

Die Erfindung betrifft weiterhin ein Verfahren zur Identifizierung von Aktivatoren eines eukaryotischen Kaliumkanals, wobei
a) eine mutierte S. cerevisiae Zelle verwendet wird, die die drei endogenen Kaliumkanäle TRK1, TRK2 und TOK1 nicht exprimiert;
b) in dieser mutierten S. cerevisiae Zelle ein eukaryotischer Kaliumkanal heterolog exprimiert wird;
c) die mutierte S. cerevisiae Zelle in Gegenwart eines Inhibitors des eukaryotischen Kaliumkanals mit einer zu untersuchenden Substanz inkubiert wird;
   und
d) der Effekt der zu untersuchenden Substanz auf den eukaryotischen Kaliumkanal bestimmt wird.

Die Erfindung betrifft auch ein Verfahren zur Herstellung eines Arzneimittels, wobei
a) ein Inhibitor eines eukaryotischen Kaliumkanals identifiziert wird ,
b) der Inhibitor nach bekannten chemischen Verfahren hergestellt oder isoliert wird, und
c) der Inhibitor mit physiologisch verträglichen Zusatzstoffen versetzt wird.

Die Erfindung betrifft auch ein Verfahren zur Herstellung eines Arzneimittels, wobei
a) ein Aktivator eines eukaryotischen Kaliumkanals identifiziert wird ,
b) der Aktivator nach bekannten chemischen Verfahren hergestellt oder isoliert wird, und
c) der Aktivator mit physiologisch verträglichen Zusatzstoffen versetzt wird.

Die Erfindung bezieht sich demnach auf folgende Gegenstände:
Verfahren zur Identifizierung von Inhibitoren eines eukaryotischen Kaliumkanals, wobei
   a) eine mutierte S. cerevisiae Zelle verwendet wird, die die drei endogenen Kaliumkanäle TRK1, TRK2 und TOK1 nicht exprimiert ;
   b) in dieser mutierten S. cerevisiae Zelle ein eukaryotischer Kaliumkanal heterolog exprimiert wird;
   c) die mutierte S. cerevisiae Zelle mit einer zu untersuchenden Substanz inkubiert wird;
      und
   d) der Effekt der zu untersuchenden Substanz auf den eukaryotischen Kaliumkanal bestimmt wird.

Verfahren, wie oben beschrieben, wobei in der mutierten S. cerevisiae Zelle die Gene TRK1, TRK2 und TOK1 ausgeschaltet sind (Δtrk1, Δtrk2, Δtok1).

Verfahren, wie oben beschrieben, wobei der eukaryotische Kaliumkanal ein humaner Kaliumkanal ist.

Verfahren, wie oben beschrieben, wobei der eukaryotische Kaliumkanal ein HERG1, Kv1.5 oder gpIRK1 ist.

Verfahren , wie oben beschrieben, wobei der eukaryotische Kaliumkanal mutiert ist.

Verfahren, wie oben beschrieben, wobei der eukaryotische Kaliumkanal in einem Hefe Expressionsplasmid vorliegt.

Verfahren , wie oben beschrieben, wobei die mutierte S. cerevisiae Zelle einen Wachstumsreporter konstitutiv exprimiert.

Verfahren, wie oben beschrieben, wobei eine zu untersuchende Substanz, die auf den eukaryotischen Kaliumkanal einen Effekt hat, das Wachstum der mutierten S. cerevisiae Zelle inhibiert.

Verfahren, wie oben beschrieben, wobei der Effekt einer zu untersuchenden Substanz auf den eukaryotischen Kaliumkanal durch Messung der Zellzahl der mutierten S. cerevisiae Zellen bestimmt wird.

Verfahren, wie oben beschrieben, wobei die Zellzahl über die Fluoreszenz oder Lumineszenz eines konstitutiv exprimierten Wachstumsreporters bestimmt wird.

Mutierte S. cerevisiae Zelle, **dadurch gekennzeichnet, daß** die endogenen Kaliumkanäle TRK1, TRK2 und TOK1 nicht exprimiert werden.

Mutierte S. cerevisiae Zelle, **dadurch gekennzeichnet, daß** die Gene TRK1, TRK2 und TOK1 ausgeschaltet sind.

Mutierte S. cerevisiae Zelle hinterlegt unter DSM 13197.

Mutierte S. cerevisiae Zelle , wie oben beschrieben, wobei die S. cerevisiae Zelle einen eukaryotischen Kaliumkanal heterolog exprimiert.

Mutierte S. cerevisiae Zelle , wie oben beschrieben, wobei der eukaryotische Kaliumkanal ein humaner Kaliumkanal ist.

Mutierte S. cerevisiae Zelle nach , wie oben beschrieben, wobei der eukaryotische Kaliumkanal ein HERG1, Kv1.5 oder gpIRK1 ist.

Mutierte S. cerevisiae Zelle , wie oben beschrieben, wobei der eukaryotische Kaliumkanal mutiert ist.

Verfahren zur Herstellung einer mutierten S. cerevisiae Zelle, die die Kaliumkanäle TRK, TRK2 und TOK1 nicht exprimiert, **dadurch gekennzeichnet, daß** die Gene TRK1, TRK2 und TOK1 durch knock out zerstört werden.

Verwendung einer mutierten S. cerevisiae Zelle , wie oben beschrieben, zur Identifizierung von Substanzen, die die Aktivität des eukaryotischen Kaliumkanals inhibieren.

Verfahren zur Identifizierung von Aktivatoren eines eukaryotischen Kaliumkanals, wobei
a) eine mutierte S. cerevisiae Zelle verwendet wird, die die drei endogenen Kaliumkanäle TRK1, TRK2 und TOK1 nicht exprimiert;
b) in dieser mutierten S. cerevisiae Zelle ein eukaryotischer Kaliumkanal heterolog exprimiert wird;
c) die mutierte S. cerevisiae Zelle mit einer zu untersuchenden Substanz inkubiert wird;
   und
d) der Effekt der zu untersuchenden Substanz auf den eukaryotischen Kaliumkanal bestimmt wird.

Verfahren zur Identifizierung von Aktivatoren eines eukaryotischen Kaliumkanals, wobei
a) eine mutierte S. cerevisiae Zelle verwendet wird, die die drei endogenen Kaliumkanäle TRK1, TRK2 und TOK1 nicht exprimiert;
b) in dieser mutierten S. cerevisiae Zelle ein eukaryotischer Kaliumkanal heterolog exprimiert wird;
c) die mutierten S. cerevisiae Zelle in Gegenwart eines Inhibitors des eukaryotischen Kaliumkanals mit einer zu untersuchenden Substanz inkubiert wird;
   und
d) der Effekt der zu untersuchenden Substanz auf den eukaryotischen Kaliumkanal bestimmt wird.

Test-Kit enthaltend eine mutierte S. cerevisiae Zelle , wie oben beschrieben.

Verfahren zur Herstellung eines Arzneimittels, wobei
a) ein Inhibitor eines eukaryotischen Kaliumkanals mit Hilfe eines wie oben beschriebenen Verfahrens zur Idenfifizierung von Inhibitoren eines eukaryontischen Kaliumkanals identifiziert wird ,
b) der Inhibitor nach bekannten chemischen Verfahren hergestellt oder isoliert wird, und
c) der Inhibitor mit physiologisch verträglichen Zusatzstoffen versetzt wird.

Verfahren zur Herstellung eines Arzneimittels, wobei
a) ein Aktivator eines eukaryotischen Kaliumkanals mit Hilfe eines wie oben beschriebenen Verfahrens zur Identifizierung von Aktivatoren eines eukaryotischen Kaliumkanals identifiziert wird ,
b) der Aktivator nach bekannten chemischen Verfahren hergestellt oder isoliert wird, und
c) der Aktivator mit physiologisch verträglichen Zusatzstoffen versetzt wird.

### Abbildungen :

Figur 1: Diagnostische PCR für die Bestätigung des Dreifach-Knockouts. Erläuterung der Reihen/Gelspuren siehe Text Beispiel 2, dreifach knock out.
Figur 2: Wachstum der Stämme YM168 (Δ*trk1* Δ*trk2*) und YM182 (Δ*trk1* Δ*trk2* Δ*tok1*) auf DPM Medium mit definierten Konzentrationen an KCl bei pH 6.5.
Figur 3: Wachstum der Stämme YM189 bzw. YM190 (in Δ*trk1* Δ*trk2*)*,* und YM194 bzw. YM195 (in Δ*trk1* Δ*trk2* Δ*tok1*) auf DPM Medium mit 5 mM KCl + 2 mM RbCl bei pH 6.5.
Figur 4: Wachstum der Stämme YM189 bzw. YM191 (in Δ*trk1* Δ*trk2*)*,* und YM194 bzw. YM196 (in Δ*trk1* Δ*trk2* Δ*tok1*) auf DPM Medium mit 5 mM KCl + 2 mM CsCl bei pH 6.5.
Figur 5: Wachstum der Stämme YM194 bzw. YM195 (in Δ*trk1* Δ*trk2* Δ*tok1)* in DPM Medium mit 5 mM KCl + 1 mM RbCl bei pH 6.5. ("KON" = Kontrolle)
Figur 6: Wachstum der Stämme YM194 bzw. YM196 (in Δ*trk1* Δ*trk2* Δ*tok1*) in DPM Medium mit 5 mM KCl + 1 mM CsCl bei pH 6.5. ("KON" = Kontrolle)
Figur 7: Expression des humanen Kaliumkanals HERG1 in der Dreifachmutante Δ*trk1*Δ*trk2*Δ*tok1* in DPM -HIS/-TRP 5mM KCl Medium in 96well ELISA-Platten in Gegenwart von 0.5 mM CsCl als Aktivator.
   Die verschiedenen Inhibitoren wurden in einer Endkonzentration von jeweils 30µM eingesetzt. Zur Messung der Zelldichte wurde ein kommerziell erhältliches LacZ-Reportersystem pYX232 der Firma Ingenius (Kat.Nr. MBV-032-10) in die zu untersuchenden Hefestämme transformiert. Die Expression des LacZ-Reportergens erfolgte unter Kontrolle des konstitutiven *Saccharomyces cerevisiae* -Promoters TPI für das Triose-Phosphat-Isomerase-Gen. Die Messung der LacZ Enzymaktivität erfolgte über Detektion der Lumineszenz nach 24h Wachstum (Dichte der Starter-Kultur: 0.01 OD₆₂₀) unter Verwendung eines kommerziell erhältlichen Assay-Systems der Firma TROPIX. Die Werte entsprechen dem Durchschnitt aus jeweils 4 Messungen ±SD. Die zwei verschiedenen Assays wurden unabhängig voneinander an zwei verschiedenen Tagen durchgeführt.
Figur 8: Expression des humanen Kaliumkanals HERG1 in der Dreifach-mutante Δ*trk*1Δ*trk*2Δ*tok*1 in DPM -HIS 5mM KCl Medium in 96well ELISA-Platten in Gegenwart von 0.5 mM CsCl als Aktivator.
   Die verschiedenen Inhibitoren wurden in einer Endkonzentration von jeweils 30µM eingesetzt. Die Messung der Zelldichte erfolgte nach 38h Wachstum (Dichte der Starter-Kultur: 0.03 OD₆₂₀) über Bestimmung der optischen Dichte bei einer Wellenlänge von 620 nm. Die Werte entsprechen dem Durchschnitt aus jeweils 4 Messungen ±SD.
Figur 9: Wachstum des *Saccharomyces cerevisiae* Wildtyp-Stamms in DPM -HIS/-TRP 5mM KCl Medium in 96well ELISA-Platten in Gegenwart von 0.5 mM CsCl.
   Die verschiedenen Inhibitoren wurden in einer Endkonzentration von jeweils 30µM eingesetzt. Zur Messung der Zelldichte wurde ein kommerziell erhältliches LacZ-Reportersystem pYX232 der Firma Ingenius (Kat.Nr. MBV-032-10) in die zu untersuchenden Hefestämme transformiert. Die Expression des LacZ-Reportergens erfolgte unter Kontrolle des konstitutiven *Saccharomyces cerevisiae* -Promoters TPI für das Triose-Phosphat-Isomerase-Gen. Die Messung der LacZ Enzymaktivität erfolgte über Detektion der Lumineszenz nach 24h Wachstum (Dichte der Starter-Kultur: 0.01 OD₆₂₀) unter Verwendung eines kommerziell erhältlichen Assay-Systems der Firma TROPIX.
   Die Werte entsprechen dem Durchschnitt aus jeweils 4 Messungen ±SD. Die zwei verschiedenen Assays wurden unabhängig voneinander an zwei verschiedenen Tagen durchgeführt.
Figur 10: Wachstum des *Saccharomyces cerevisiae* Wildtyp-Stamms in DPM -Medium in 96well ELISA-Platten in Gegenwart von 5mM KCl oder in Gegewart von 80mM KCl. Die Inhibitoren Ziprasidon und Pimozid wurden in einer Endkonzentration von jeweils 30µM eingesetzt. Die Messung der Zelldichte erfolgte nach 24h Wachstum (Dichte der Starter-Kultur: 0.01 OD₆₂₀) über Bestimmung der optischen Dichte bei einer Wellenlänge von 620 nm. Die Werte entsprechen dem Durchschnitt aus jeweils 4 Messungen ±SD.
Figur 11: Expression des humanen Kaliumkanals HERG1 in der Dreifach-mutante Δ*trk*1Δ*trk*2Δ*tok*1*,* sowie in der Doppelmutante Δ*trk*1Δ*trk*2 auf DPM -HIS Medium in Gegenwart von 5mM KCl und 0.5 mM CsCl als Aktivator.
   1: Wachstum der Dreifach-Mutante Δ*trk*1Δ*trk*2Δ*tok*1 bei Expression des Leervektors p423GPD als negative Kontrolle. 2: Wachstum der Dreifach-Mutante Δ*trk*1Δ*trk*2Δ*tok*1 bei Expression von p423GPD-TRK1 als positive Kontrolle. 3: Wachstum der Dreifach-Mutante Δ*trk*1Δ*trk*2Δ*tok*1 bei Expression von p423GPD-HERG1. 4: Wachstum der Doppel-Mutante Δ*trk*1Δ*trk*2 bei Expression von p423GPD-HERG1. Die verwendeten Vektoren und Konstrukte sind in der Patentanmeldung erläutert (s. Seite 12ff und 15ff).
Figur 12: Expression des humanen Kaliumkanals Kv1.5 in der Dreifach-mutante Δ*trk*1Δ*trk*2Δ*tok*1*,* sowie in der Doppelmutante Δ*trk*1Δ*trk*2 auf DPM -HIS Medium in Gegenwart von 5mM KCl und 2 mM RbCl als Aktivator.
   1: Wachstum der Dreifach-Mutante Δ*trk*1c*trk*2Δ*tok*1 bei Expression des Leervektors p423GPD als negative Kontrolle. 2: Wachstum der Dreifach-Mutante Δ*trk*1Δ*trk*2Δ*tok*1 bei Expression von p423GPD-TRK1 als positive Kontrolle. 3: Wachstum der Dreifach-Mutante Δ*trk*1Δ*trk*2Δ*tok*1 bei Expression von p423GPD-Kv1.5. 4: Wachstum der Doppel-Mutante Δ*trk*1Δ*trk*2 bei Expression von p423GPD-Kv1.5. Die verwendeten Vektoren und Konstrukte sind in der Patentanmeldung erläutert (s. Seite12ff und 15ff).
Figur 13: Expression des humanen Kaliumkanals ROMK2, bzw. des Hefevektors p423GPD als Negativkontrolle in der Dreifach-mutante Δ*trk*1Δ*trk*2Δ*tok*1 in DPM -HIS 5mM KCl Medium in 96well ELISA-Platten.
   Die Messung der Zelldichte erfolgte nach 24h Wachstum (Dichte der Starter-Kultur: 0.01 OD₆₂₀) über Bestimmung der optischen Dichte bei einer Wellenlänge von 620 nm. Die Werte entsprechen dem Durchschnitt aus jeweils 4 Messungen ±SD.
Figur 14: Plasmid Karte von p423 GPD-ROMK2.

### Beispiele:

### Material und Stämme

### Medien

YPD (Hefe-Vollmedium): 1% Bacto Yeast Extract, 2% Bacto-Pepton, 2% Bacto-Agar, 2% Glucose.
SC (synthetic complete) Medium: 0.67% Bacto-Yeast Nitrogen Base, Aminosäuren, 2% Glucose.
Sporulationsmedium: 1 % Kaliumacetat, Aminosäuren.
5-FOA Medium: 0.67% Bacto-Yeast Nitrogen Base, Aminosäuren, Uracil (50 µg/ml), 2% Zucker (Galactose oder Glucose), 0,1% 5-FOA
Alle Medien sind beschrieben in: (Fink, G. R. et al., 1991)

Aminosäure Dropout-Mix:
L-Alanin 2 g; L-Arginin 2 g; L-Asparagin*H₂O 2.27 g; L-Asparaginsäure 2 g;
L-Cystein*HCl 2.6 g; L-Glutamin 2 g; L-Glutaminsäure 2 g; Glycin 2 g; myo-Inositol 2 g; L-Isoleucin 2 g; L-Methionin 2 g; PABA 0.2 g; L-Phenylalanin 2 g; L-Prolin 2 g;
L-Serin 2 g; L-Threonin 2 g; L-Tyrosin 2 g; L-Valin 2 g.

Stocklösungen für Marker-Aminosäuren:

| | mM | g/l | |
|---|---|---|---|
| Adenin (100x) | 30 | 5.53 | erwärmen (bis max.60°C) |
| Leucin (60x) | 100 | 13.12 | erwärmen |
| Lysin (100x) | 100 | 18.26 | - |
| Histidin (200x) | 60 | 12.57 | - |
| Tryptophan (100x) | 40 | 8.17 | - |
| Uracil (100x) | 20 | 2.24 | in 0.5% NaHCO₃-Lösung erwärmen |

Vitamin Stock (50 ml): Biotin 20 µg/l; Ca-Pantothenate 40 µg/l; Thiamine 40 µg/l.

Defined potassium medium (DPM): für 1.5 I (2x-Stock):

| | | |
|---|---|---|
| (NH₄⁾₂HPO₄ | 8mM | 3.2 g |
| (NH₄⁾₂SO₄ | 29 mM | 11.5 g |
| MgSO₄ | 2 mM | 0.8 g (bzw. 6 ml von 1 M Stock) |
| CaCl₂ | 0.2 mM | 90 µg (bzw. 1.2 ml von 0.5 M Stock) |
| Vitamine Stock | | 120 µl |
| Aminosäure Dropout-Mix | | 6 g |
| Lysin | | 330 ml von 100x Stock |
| Adenin | 0.9 mM | 30 ml von 100x Stock |
| → mit HCl pH 6.5 (bzw. einen anderen pH-Wert) einstellen, autoklavieren | | |
| Glucose | 2 % | von 40 % Stock |
| KCl | | von 1 M Stock |
| essentielle Aminosäuren (außer Lys/Ade)von Stocks | | |
| Agar | | |

Puffer und Lösungen:
TE-Puffer: Tris/HCl (pH 7.5) 10 mM; EDTA (pH 8.0)1 mM;
TAE-Puffer: Tris 40 mM; EDTA 1 mM; Essigsäure 0.2 mM;
SSC-Puffer (20x): NaCl 3M; Natriumcitrat*2 H₂O 0.3 M;
Gel-Ladepuffer: Bromphenolblau 0.05% (w/v); Sucrose 40% (w/v); EDTA, pH 8.0 0.1 M; SDS 0.5% (w/v);
Hybridisationspuffer: SSC 5x; SDS 0.1% (w/v); Dextransulfat 5% (w/v); Stopreagenz 1:20;
Puffer A (steril): Tris-HCl 100 mM; NaCl, pH 9.5 300 mM;
Depurinationslösung: HCl 0.25 M;
Denaturierungslösung: NaCl 1.5 M; NaOH 0.5 M;
Neutralisationslösung: NaCl 1.5 M; Tris, pH 8.0 0.5 M.

Oligonukleotide (Primer für PCR's):

| Name | Sequenz (5'→ 3') | RE |
|---|---|---|
| TRK1-FL-BamHl- | SEQ ID NO. 7: | *Bam*H |
| Fo | GCG'GATCCATGCATTTTAGAAGAACGATGAGTAG | I |
| TRK1-FL-Pstl-Re | SEQ ID NO. 8: | *Pst*I |
| | AGGTTCTGCTGCA'GTTGGTGT | |
| TRK1-FL-Pstl-Fo | SEQ ID NO. 9: | *Pst*I |
| | ACACCAACTGCA'GCAGAACCT | |
| TRK1-FL-Xhol-Re | SEQ ID NO. 10: | *Xho*I |
| | CGC'TCGAGTTAGAGCGTTGTGCTGCTCCT | |
| TRK1-Dia-Fo | SEQ ID NO. 11: | --- |
| | CCTTACCATTAGCATCACTGAT | |
| TRK1-Dia-Re1 | SEQ ID NO. 12: | --- |
| | CTATTAACCATTTCTCCGCTG | |
| URA-Rev | SEQ ID NO. 13: | --- |
| | GATTTATCTTCGTTTCCTGCAGGT | |
| TRK2-DEL-5-Fo-B | SEQ ID NO. 14: | *Bsi*WI |
| | CAC'GTACGTCCAGCACAATTTCACAACAGCT | |
| TRK2-DEL-5-Re | SEQ ID NO. 15: | *Sal*I |
| | CAG'TCGACCTGGATGACGTCCTCTTAGCTG | |
| TRK2-DEL-3-Fo | SEQ ID NO. 16: | *Eco*R |
| | CAGAT'ATCATGCTGCCAAGTGACAAACTG | V |
| TRK2-DEL-3-Re | SEQ ID NO. 17: | *Spe*I |
| | TCA'CTAGTTGTTGATGGCTTTGGTTGGT | |
| TRK2-Dia-Fo | SEQ ID NO. 18: | --- |
| | GCGAAGAATAGGATGAGATGTG | |
| TRK2-Dia-Re1 | SEQ ID NO. 19: | --- |
| | TTGTCGTGGGTCTTCTCTGG | |
| KAN-Rev | SEQ ID NO. 20: | --- |
| | GCTACCTTTGCCATGTTTCAGAA | |
| TOK1-DEL-5-Fo | SEQ ID NO. 21: | *Bsi*WI |
| | CAC'GTACGGCAAATTTATCGAGACTCTGCGA | |
| TOK1-DEL-5-Re | SEQ ID NO. 22: | *Sal*I |
| | AGG'TCGACCATATTGCCATATCCCAGCGT | |
| TOK1-DEL-3-Fo | SEQ ID NO. 23: | *Eco*R |
| | TGGAT'ATCACCTGATACGCCC | V |
| TOK1-DEL-3-Re | SEQ ID NO. 24: | *Spe*I |
| | CAA'CTAGTGCATACCAGTAGTATGAGACATGCTT G | |
| TOK1-Dia-Fo | SEQ ID NO. 25: | --- |
| | CCTGAGTACTCAGTACCATCTTG | |
| TOK1-Dia-Re1 | SEQ ID NO. 26: | --- |
| | CTGTAGATGCTGGGCATG | |
| Kv1.5-GFP-Fo | SEQ ID NO. 27: | *Sal*I |
| | TACG'TCGACATGGAGATCGCCCTGGTG | |
| Kv1.5-GFP-Re | SEQ ID NO. 28: | *Sal*I |
| | TACG'TCGACATCTGTTTCCCGGCTGGTG | |
| HERG1-GFP-Fo | SEQ ID NO. 29: | *Cla*I |
| | TACAT'CGATATGCCGGTGCGGAGGG | |
| HERG1-GFP-Re | SEQ ID NO. 30: | *Sal*I |
| | TACG'TCGACACTGCCCGGGTCCGA | |

### Vektoren:

### Bakterielle Vektoren

| Name | Größe (bp) | Gene |
|---|---|---|
| pcDNA3 (*Invitrogen*) | 5446 | CMV-Prom., T7-Prom., Polylinker, Sp6-Prom., BGH poly (A), SV40 Prom., SV 40 ori, Neomycin^{R}, SV 40 poly (A), ColE1 ori, Amp^{R} |
| pcDNA3.1 (+/-) (*Invitrogen*) | 5432 | CMV-Prom., T7-Prom./priming site, MCS, pcDNA3.1 reverse priming site, BGH poly (A), F1 ori, SV40 Prom., SV 40 ori, Neomycin^{R}, SV 40 poly (A), ColE1 ori, Amp^{R} |
| pUG6 | 4009 | *IoxP*-TEF2-Prom.-kanMX-*IoxP*-TEF2-Term., ori, Amp^{R} |
| pCR®-Blunt II-TOPO | 3519 | *lac*-Prom./Op., M13 Reverse prim. site, LacZ-α ORF, SP6-Prom. prim. site, MCS, TOPO™-Cloning site, T7 Prom. prim. site, M13 (-20) Forward prim. site, M13 (-40) prim. site, Fusion point, *ccdB* lethal gene ORF, *kan* gene, (*kan*-Prom., Kanamycin resistance gene ORF), Zeocin resistence ORF, pMB1 origin (pUC-derived) |
| pCR® II-TOPO | 3900 | LacZ-α gene, M13 Reverse prim. site, SP6-Prom., MCS, T7-Prom., M13 (-20) Forward prim. site, M13 (-40) Forward prim. site, f1 origin, Kanamycin resistance ORF, Ampicillin resistence ORF, pMB1 origin (pUC-derived) |

### Hefe Vektoren

| Name | Größe (bp) | Gene |
|---|---|---|
| pSH47 | 6786 | CEN6/ARSH4, URA3, CYC1-Term., CRE, GAL1-Prom., Amp |
| p414 GAL1 | 5474 | CEN6/ARSH4, TRP1, CYC1-Term., GAL1-Prom., Amp^{R} |
| p416 GAL1 | 5584 | CEN6/ARSH4, URA3, CYC1-Term., GAL1-Prom., Amp^{R} |
| p416 ADH | 6624 | CEN6/ARSH4, URA3, CYC1-Term., ADH-Prom., Amp^{R} |
| p423 GPD3 | 6678 | 2µ, HIS3, CYC1-Term., GPD3-Prom., Amp^{R} |
| p426 GAL1 | 6417 | 2µ, URA3, CYC1-Term., GAL1-Prom., Amp^{R} |
| p426 GAL1-yEGFP3 | 7140 | 2µ, URA 3, CYC1-Term., yEGFP3, GAL1-Prom., Amp^{R} |
| p426 GAL1-SP- | 7227 | 2µ, URA 3, CYC1-Term., N-terminal 24 aa von Ste2, |
| yEGFP3 | | yEGFP3, GAL1-Prom., Amp^{R} |

Stämme:
Bakterienstämme: DH5α; One Shot™ TOP10 (*Invitrogen*)

Hefestämme:
Alle für diese Arbeit generierten Hefestämme basieren auf dem diploiden Wildtypstamm:
W303 MATa/α ade2, his3-11-15, leu2-3-112, trp1-1, ura3-1, can1-100; ATCC-Nr. 208352.

| Stamm | Urspr. Name | Mating-typ | Gene |
|---|---|---|---|
| YM 96 | w303 | MATa/α | ade2, his3-11-15, leu2-3-112, trp1-1, ura3-1, can1-100 |
| YM 97 | w303 | MATa | ade2, his3-11-15, leu2-3-112, trp1-1, ura3-1, can1-100 |
| YM 98 | w303 | MATα | ade2, his3-11-15, leu2-3-112, trp1-1, ura3-1, can1-100 |

Die folgenden Hefestämme wurden generiert:

| Stamm | Urspr. Name | Mating- | Gene (außer ade2, his3-11-15, leu2-3-112, |
|---|---|---|---|
| | | typ | trp1-1, ura3-1, can1-100) |
| YM 123 | Δtrk1 in YM 96 | MATα | trk1::*his*G-URA3-*his*G |
| YM 124 | Δtrk1 in YM 96 | MATa | trk1::*his*G-URA3-*his*G |
| YM 139 | Δtok1 in YM 96 | MATa/α | tok1::*Iox*P-KanMX-*Iox*P |
| YM140 | Δtok1 in YM 123 | MATα | trk1::*his*G-URA3-*his*G, tok1::*Iox*P-KanMX-*Iox*P |
| YM 141 | Δtok1 in YM 123 | MATα | trk1::*his*G-URA3-*his*G, tok1::*Iox*P-KanMX-*IoxP* |
| YM 142 | Δtok1 in YM 96 | MATa/α | tok1::*Iox*P-KanMX-*Iox*P |
| YM 143 | Δtok1 in YM 124 | MATa | trk1::*his*G-URA3-*his*G, tok1::*Iox*P-KanMX-*IoxP* |
| YM144 | Δtok1 in YM 124 | MATa | trk1::*his*G-URA3-*his*G, tok1::*Iox*P-KanMX-*IoxP* |
| YM 154 | Δtok1 in YM 96 | MATα | tok1::*Iox*P-KanMX-*Iox*P |
| YM 155 | Δtok1 in YM 96 | MATa | tok1::*Iox*P-KanMX-*Iox*P |
| YM 156 | Δtok1 in YM 96 | MATa | tok1::*Iox*P-KanMX-*Iox*P |
| YM 157 | Δtok1 in YM 96 | MATα | tok1::*Iox*P-KanMX-*Iox*P |
| YM 158 | Δtrk2 in YM 96 | MATα | trk2::*Iox*P-KanMX-*Iox*P |
| YM 159 | Δtrk2 in YM 96 | MATa | trk2::*Iox*P-KanMX-*Iox*P |
| YM 160 | Δtrk2 in YM 96 | MATa | trk2::*Iox*P-KanMX-*Iox*P |
| YM 161 | Δtrk2 in YM 96 | MATα | trk2::*Iox*P-KanMX-*Iox*P |
| YM 162 | Δtok1 in YM 123 | MATα | trk1::*his*G, tok1::*Iox*P |
| YM 163 | Δtok1 in YM 123 | MATα | trk1::*his*G, tok1::*Iox*P |
| YM 164 | Δtok1 in YM 124 | MATa | trk1::*his*G, tok1::*Iox*P |
| YM 165 | Δtok1 in YM 124 | MATa | trk1::*his*G, tok1::*Iox*P |
| YM 166 | YM 124 x YM 160 | MATa | trk1::*his*G-URA3-*his*G, trk2::*Iox*P-KanMX-*IoxP* |
| YM 167 | YM 124 x YM 160 | MATa | trk1::*his*G-URA3-*his*G, trk2::*Iox*P-KanMX-*Iox*P |
| YM 168 | YM 124 x YM 160 | MATα | trk1::*his*G-URA3-*his*G, trk2::*Iox*P-KanMX-*Iox*P |
| YM 169 | YM 124 x YM 160 | MATα | trk1::*his*G-URA3-*his*G, trk2::*Iox*P-KanMX-*Iox*P |
| YM 182 | Δtrk2 in YM 165 | MATa | trk1::*his*G, tok1::*Iox*P, trk2::*Iox*P-KanMX-*Iox*P |
| YM 183 | YM 166 | MATa | trk1::*his*G, tok1::*Iox*P, trk2::*Iox*P-KanMX-*Iox*P |
| YM 184 | YM 168 | MATa | trkl::*his*G, tok1::*Iox*P, trk2::*Iox*P-KanMX-*Iox*P |
| YM 185 | Kv1.5-pRS426-Gal1-yEGFP3 in YM 97 | MATa | pRS426-GAL1 mit Kv1.5-GFP3, trk1::*his*G, tok1::*Iox*P, trk2::*Iox*P-KanMX-*Iox*P |
| YM 186 | Kv1.5-pRS426-Gal1-SP-yEGFP3 in YM 97 | MATa | pRS426-GAL1 mit N24 Ste2-Kv1.5-GFP3, trk1::*his*G, tok1::*Iox*P, trk2::*Iox*P-KanMX-*Iox*P |
| YM 187 | Kv1.5-pRS426-Gal1-yEGFP3 in YM 182 | MATa | pRS426-GAL1 mit Kv1.5-GFP3, trk1::*his*G,tok1::*Iox*P, trk2::*Iox*P-KanMX-*Iox*P |
| YM 188 | Kv1.5-pRS426-Gal1-SP-yEGFP3 in YM 182 | MATa | pRS426-GAL1 mit N24 Ste2-Kv1.5-GFP3, trk1::*his*G, tok1::*Iox*P, trk2::*Iox*P-KanMX-*Iox*P |
| YM 189 | p423-GPD3 in YM 168 | MATa | p423-GPD3, trk1::*his*G-URA3-*his*G, trk2::*Iox*P-KanMX-*Iox*P |
| YM 190 | Kv1.5-p423-GPD3 in YM 168 | MATa | p423-GPD3 mit Kv1.5, trk1::*his*G-URA3-*his*G, trk2::*Iox*P-KanMX-*Iox*P |
| YM 191 | HERG-p423-GPD3 in YM 168 | MATa | p423-GPD3 mit HERG, trk1::*his*G-URA3-*his*G, trk2::*Iox*P-KanMX-*Iox*P |
| YM 192 | HCN2-p423-GPD3 in YM 168 | MATa | p423-GPD3 mit HCN2, trk1::*his*G-URA3-*his*G, trk2::*Iox*P-KanMX-*Iox*P |
| YM 193 | IRK1-p423-GPD3 in YM 168 | MATa | p423-GPD3 mit IRK1, trk1::*his*G-URA3-*his*G, trk2::*Iox*P-KanMX-*Iox*P |
| YM 194 | p423-GPD3 in YM 182 | MATa | p423-GPD3, trk1::*his*G, tok1::*Iox*P, trk2::*Iox*P-KanMX-*Iox*P |
| YM 195 | Kv1.5-p423-GPD3 in YM 182 | MATa | p423-GPD3 mit Kv1.5, trk1::*his*G, tok1::*Iox*P, trk2::*Iox*P-KanMX-*Iox*P |
| YM 196 | HERG-p423-GPD3 in YM 182 | MATa | p423-GPD3 mit HERG, trk1::*his*G, tok1::*Iox*P, trk2::*Iox*P-KanMX-*Iox*P |
| YM 197 | HCN2-p423-GPD3 in YM 182 | MATa | p423-GPD3 mit HCN2, trk1::*his*G, tok1::*Iox*P, trk2::*Iox*P-KanMX-*Iox*P |
| YM 198 | IRK1-p423-GPD3 in YM 182 | MATa | p423-GPD3 mit IRK1, trk1::*his*G, tok1::*Iox*P, trk2::*Iox*P-KanMX-*Iox*P |
| YM 199 | TRK1-p423-GPD3 in YM 182 | MATa | p423-GPD3 mit TRK1, trk1::*his*G, tok1::*Iox*P, trk2::*Iox*P-KanMX-*Iox*P |

Klonierte Kaliumkanäle:

| A) | |
|---|---|
| systematischer Name | KCNA5 |
| Synonyme | Kv1.5, (HK2, HPCN1) |

| | |
|---|---|
| Familie | spannungsgesteuerter Kaliumkanal, Shaker verwandte Subfamilie (Mitglied Nr.5), verzögerter Gleichrichter (delayed rectifier) |
| chromosomale Lokalisation | 12p13.32-p13.31 |
| Accession | NID g4504818 |
| Protein | 613 As, 67 kD |
| Vorkommen im Gewebe | Herz, pankreatische Inseln und Insulinoma |
| Homologe | mKcna5 (*Mus musculus*)*,* 70% zu hHCN4 |
| Referenzen | (Roberds, S. L. et al., 1991; Curran, M. E. et al., 1992; |
| Snyders, D. J. et al., 1993) | |

| | |
|---|---|
| B) | |
| systematischer Name | HCN2 |
| Synonyme | BCNG2 (Brain cyclic nucleotide gated channel), HAC1 |
| Familie | durch Hyperpolarisation aktiviert und durch zyklische Nukleotide gesteuerter Kaliumkanal, gehört zur Superfamilie der spannungsgesteuerten Kaliumkanäle |
| chromosomale Lokalisation | 19p13.3 |
| Accession | NID g4996893 g4775348 |
| Protein | 889 As |
| Funktion | Schrittmacher |
| Vorkommen im Gewebe | Gehirn, Herz |
| Homologe | mHcn2 (*Mus musculus*) |
| Referenzen | (Ludwig, A. et al., 1999) |

| | |
|---|---|
| C) | |
| systematischer Name | KCNH2 |
| Synonyme | HERG1 (longer splice variant) |
| Familie | spannungsgesteuerter Kaliumkanal, eag verwandte Subfamilie, Mitglied Nr.2 |
| chromosomale Lokalisation | 7q35-q36 |
| Accession | NID g4557728 g4156210 |
| Eigenschaften | Kanalaktivierung durch K⁺-Kanal Regulator 1 beschleunigt |
| Referenzen | (Taglialatela, M. et al., 1998; Itoh, T. et al., 1998) |

| D) | |
|---|---|
| systematischer Name | KCNJ2 (guinea pig) |
| Synonyme | Kir2.1, IRK1 |
| Familie | einwärtsgleichrichtender Kaliumkanal |
| Vorkommen im Gewebe | Gehirn, Herz, Lunge, Niere, Placenta, Skelettmuskulatur |
| Referenzen | (Tang, W. et al., 1995) |

Methoden:
ROMK2 (s. Anlage "Sequenz ROMK2")

PCR:
Protokoll für Powerscript Polymerase (*PAN Biotech*)*:*
Mix für unteres Reagenz (Hot Start -Protokoll) (25 µl):
   3 µl H₂O; 2.5 µl 10x OptiPerform™ III-Puffer, pH 9.2; 10 µl 1.25 mM dNTP's (= 200 µM);
   1.5 µl Primer Forward (20 pmol/µl); 1.5 µl Primer Reverse (20 pmol/µl); 1.5 µl 50 mM MgCl₂ (= 1.25 mM); 5 µl 5x OptiZyme™ Enhancer.
Mix für oberes Reagenz (35 µl):
   23 µl H₂O; 3.5 µl 10x OptiPerform™ III-Puffer; 1.5 µl 50 mM MgCl₂, 0.5 µl PowerScript DNA Polymerase; 7 µl 5x OptiZyme™ Enhancer.

PCR-Programm (Hot-Start):
1. 94°C für 1 min
2. 94°C für 1 min
3. 50-55°C (je nach Primer) für 1.5 min
4. 69-72°C (je nach Polymerase) für 4 min
5. zurück zu 2., 27x wiederholen
6. 4°C ∞
7. Ende.

Protokoll für AmpliTaq Polymerase (*Perkin Elmer*)*:*
Mix für oberes Reagenz (Hot Start -Protokoll) (50 µl):
   18.1 µl H₂O; 4.2 µl 10x Puffer II; 16.7 µl dNTPs; 2.5 µl Primer Forward; 2.5 µl Primer Reverse; 6 µl 25 mM MgCl₂ (= 1.5 mM).
Mix für unteres Reagenz (50 µl):
   42 µl H₂O; 5 µl 10x Puffer II; 1 µl AmpliTaq Polymerase; 2 µl Template.

### DNA-Reinigung

Reinigung von PCR-Reaktionsansätzen: Die Reinigung von PCR-Amplifikationsprodukten erfolgte mit dem High Pure PCR Product Purification Kit (Roche)

Phenolextraktion: Probenvolumen mit TE-Puffer auf 200 µl auffüllen. Zugabe von 200 µl Phenol/Chloroform/Isoamylalkohol (25:24:1), mischen und 1 min bei maximaler Drehzahl zentrifugieren. Obere Phase in neues Eppendorfgefäß überführen, Zugabe von 200 µl Chloroform/Isoamylalkohol, mischen, 1 min zentrifugieren. Obere Phase abnehmen, anschließend Ethanolfällung.
Ethanolfällung: 5 µl 5 M NaCl und 20 µl 3 M NaAc (pH 5.7) zu einem Probenvolumen von ca. 200µl pipettieren. Das 2,5-fache Volumen an 100% Ethanol zugeben, mischen und mindestens 30 min oder länger bei - 20°C lagern, bei 4°C 10 min zentrifugieren, Pellet in 170 µl 70% kaltem Ethanol waschen, 3 min zentrifugieren und Pellet bei 37°C trocknen und in 30 µl H₂O resuspendieren.
Reinigung von Plasmid-DNA aus *E.coli:* Die Reinigung von Plasmid-DNA aus *E.coli-*Übernachtkulturen erfolgte mit dem *QIAprep Spin Miniprep Kit Protocol (Qiagen)*

DNA-Präparation aus Saccharomyces cerevisiae:
Die Hefezellen über Nacht bei 30°C in 10 ml YPD inkubieren, morgens: Zentrifugieren für 10 min bei 3000 Rpm und Pellet in 500 µl 1 M Sorbitol, 0.1 M EDTA (pH 7.5) resuspendieren und in ein Eppendorfröhrchen überführen.Zugabe von 50 µl Zymolase (5 mg/ml, in Sorbitol/EDTA)und 1 h bei 37°C inkubieren,1 min zentrifugieren. Das Pellet in 500 µl 50 mM Tris, 20 mM EDTA (pH 7.4) resuspendieren. Zugabe von 50 µl 10% SDS, gut mischen und 30 min bei 65°C inkubieren, Zugabe von 200 µl 5 M KAc, 1 h auf Eis stellenund 10 min zentrifugieren. Den Überstand (ca.650 µl) in ein neues Eppendorfröhrchen überführen und 1 Vol Isopropanol zugeben leicht mischen und 5 min stehenlassen. Entweder kurz abzentrifugieren oder ausgefallene DNA mit Glashäkchen rausziehen und Pellet lufttrocknen lassen. Das Pellet bzw. DNA in 150 µl TE-Puffer resuspendieren, für 10 min bei 65°C lösen.

DNA-Klonierungstechniken: Sämtliche DNA-Klonierungstechniken wurden gemäß Standardprotokollen durchgeführt.

### Hefetransformation (Lithium-Acetat-Methode):

Den zu transformierenden Hefestamm in 5 ml geeignetes Medium über Nacht bei 30°C auf dem Schüttler inkubieren; morgens Verdünnen der Übernachtkultur mit geeignetem Medium (OD₆₀₀ = 0.4-0.5) und Inkubation für weitere 2 h auf dem Schüttler bei 30°C (OD₆₀₀ = 0.4-0.8). Zentrifugation für 3 min bei 2500 rpm und Pellet mit 25 ml sterilem H₂O waschen, Zentrifugation für 3 min bei 2500 rpm; Pellet in 1 ml LITE (100 mM LiAc, TE pH 7.5) resuspendieren und in Eppendorfgefäß überführen. 5 min bei RT inkubieren, Zentrifugation für 15 sec (Quickspin); Pellet mit 1 ml 100 mM LiAc waschen, Quickspin; Pellet je nach Zelldichte mit 200-400 µl 100 mM LiAc resuspendieren und Aliquotieren in 50 µl-Aliquots

In der exakten Reihenfolge zugeben:
240 µl PEG (50%), Suspension durch sanftes Pipettieren mischen
36 µl 1 M LiAc, Suspension durch sanftes Pipettieren mischen
10 µl ss-sperm DNA (bei -20°C gelagert, vor Gebrauch 10 Minuten bei 80-90°C erhitzen und dann auf Eis)
2-3 µg Plasmid DNA (bzw. 8-10 µl von Miniprep, wenn Knockout-Transformation), Suspension durch sanftes Pipettieren mischen
Transformationsansatz für 30 min bei 30°C im Überkopf-Rotator bei langsamer Drehzahl inkubieren
Transformationsansatz für 15 min bei 42°C
Quickspin, Pellet in 200 µl TE-Puffer resuspendieren (bei Knockout: Pellet in 300 µl YPD resuspendieren und 4 h bei 30°C im Überkopf-Rotator inkubieren)
pro Agarplatte je 100 µl (bei Knockout den gesamten Ansatz) ausplattieren und Inkubation für 3-4 d bei 30°C

Sequenzierung: ABI PRISM™Red.Protokoll/AmpliTaq^{®}FS ¼ BigDyeTerminator

Reaktionsansatz:

| | | |
|---|---|---|
| Premix | | 2 µl |
| DNA-Template | | |
| | SsDNA | 50 ng |
| | DsDNA | 250 ng |
| | PCR-Produkte (0.2-5 kB) | 10 - 50 ng |
| Primer | | 3-10 pmol |
| H₂O bis zu Endvolumen | | 10 µl |

Thermocycler Protokoll (25 Zyklen):
1. 96°C für 15 s
2. 96°C für 15 s
3. 55°C für 10 s
4. 60°C für 4 min
5. zurück zu 2., 24x
6. 4°C ∞
7. Ende.

Aufreinigung Reaktionsansatz (Centri Sep Spin Colums, *Princeton Separations*): Säule mit 750 µl H₂O 30 min vorquellen; Flüssigkeit ablaufen lassen; 2 min bei 3000 rpm zentrifugieren; Reaktionsansatz mit H₂O auf 20 µl auffüllen und auf Säule geben; 2 min bei 3000 rpm zentrifugieren.

Probenauftrag: in Sequenziertubes 4 µl Centri Sep Eluat + 20 µl TSR (Template Suppression Reagent); 2 min bei 90°C denaturieren.

Southern Blot:
DNA-Sonde mit entsprechenden Restriktionsenzymen verdauen, mittels Gelelektrophorese auftrennen und aus dem Gel extrahieren. Ggenomische DNA über Nacht mit entsprechenden Restriktionsenzymen verdauen und mittels Gelelektrophorese (1 % Agarosegel) auftrennen

Gel-Vorbehandlung: Taschen des Agarosegels abtrennen. Agarosegel 15 min in 0.25 M HCl depurinieren, anschließend 2x in Aqua dest. waschen; Agarosegel 30 min in 0.5 M NaOH denaturieren; Transfer mit dem Vakuum Blotter Model 785 (*BioRad*): ein Fenster in die Mitte des Vinylblattes schneiden (Fensterdichtung), am Rand jeweils 0.5 cm kleiner als das Gel Nylonmembran und Filterpapier zurechtschneiden, am Rand jeweils 0.5 cm größer als das Fenster im Vinylblatt und Nylonmembran in Aqua dest. anfeuchten, anschließend Nylonmembran und Filterpapier mit Transferlösung anfeuchten.

Aufbau der Apparatur (von unten nach oben):
Basiseinheit, Vakuumbühne, poröse Vakuumplatte, Filterpapier, Nylonmembran, Vinylfenster, Agarosegel, Abschlußrahmen, Deckel
BioRad Vakuumpumpe 10 min vorwärmen, Vakuum anlegen (5 inches Hg) Gel am Rand leicht andrücken
Transferlösung (ca.1 I 10x SSC) in oberes Reservoir geben;Transfer für 90 min Vakuum ausschalten, Nylonmembran entfernen und in 2x SSC für 5 min spülen, anschließend zwischen Filterpapier lufttrocknen lassen DNA-Immobilisierung: Nylonmembran auf UV-durchläßige Klarsichtfolie legen und am Rand als positive Kontrolle Sonde aufgeben; in den UV-Stratalinker legen und Crosslinking starten (1200000 J → 0); Lagerung der Membran in Klarsichtfolie oder zwischen Whatman-Filterpapier bei Raumtemperatur bzw.4°C möglich.

*Gene Images* Random Prime Labelling Module (*Amersham*):
Markierung der DNA-Sonde: DNA-Sonde 5 min bei 96°C denaturieren (Hitzeschock), danach auf Eis.10 µl Reaktionsmix (Nukleotidmix(5x, Fluorescein-11-dUTP, dATP, dCTP, dGTP und dTTP in Tris-HCl, pH 7.8, 2-Mercaptoethanol und M₉Cl₂), 5 µl Primer (Random Nonamers); 1 µl Enzymlösung (Klenow-Fragment, 5 units/ml); 22 µl denaturierte DNA-Sonde; 12 µl H₂O. 2 h bei 37°C inkubieren und 2 µl 0.5 M EDTA (=20 mM) zugeben, Aliquiots bei -20°C lagern Überprüfung der Markierungseffizienz: 5x Nukleotidmix mit TE-Puffer 1/5, 1/10, 1/25, 1/50, 1/100, 1/250 und 1/500 verdünnen; 5 µl DNA-Sonde mit 5 µl 1/5-Verdünnung auf einen Nylonmemranstreifen auftragen, kurz absorbieren lassen und 15 min bei 60°C in vorgewärmten 2x SSC waschen; die restlichen Verdünnungen, ohne die 1/5-Verdünnung auf einem Referenzmembranstreifen auftragen und beide Membranstreifen unter UV-Licht betrachten → Bestimmung der Probenintensität.

Hybridisierung: Nylonmembran (Blot) mit erwärmten Hybridisierungsbuffer (0.3 ml/cm²) für 2 h bei 60°C im Drehofen vorhybridisieren; Puffer abgießen, davon 10 ml zurückhalten; DNA-Sonde (20 µl) denaturieren (5 min bei 96°C, danach auf Eis kühlen); Sonde mit den 10 ml Puffer auf den Blot geben und über Nacht im Drehofen bei 60°C hybridisieren.

Waschschritte:
15 min auf Platform-Schüttler in erwärmten 1x SSC, 0.1 % (w/v) SDS; 15 min auf Platform-Schüttler in erwärmten 0.5x SSC, 0.1 % (w/v) SDS

Gene Images CDP-*Star* Detection Module (Amersham):
Abstop- und Antikörperreaktion: Blot bei Raumtemperatur 1 h in einer 1/10-Verdünnung Stopreagenz in Puffer A auf Schüttler inkubieren;
Antikörperlösung (an Anti-Fluorescein gekoppelte Alkalische Phosphatase, 5000x) mit 0.5% (w/v) BSA/Puffer A verdünnen, mit Blot in Folie einschweißen und bei Raumtemperatur 1 h auf Schüttler inkubieren; ungebundene Antikörperlösung durch 3x 10 min Waschen in 0.3% Tween 20 in Puffer A entfernen
   Signalerzeugung und Detektion: Waschpuffer abgießen, Blot auf Klarsichtfolie legen; 5 ml Detektionsreagenz aufgeben, 2-5 min reagieren lassen und wieder abgießen (durch die Alkalische Phosphatase kommt es zu einer Lichtproduktion); in Klarsichtfolie einwickeln und in Dunkelkammer bei Rotlicht Film (Hyperfilm™ MP, *Amersham*) auflegen, 0.5-2 h in Filmkassette (BioMax, *Kodak*) wirken lassen, entwickeln und scannen; Blot kann bei 4°C in Klarsichtfolie gelagert werden.

### Beispiel 1: Konstruktion der spezifischen Deletionskassetten

Alle Deletionen wurden nach Standardmethoden durchgeführt (Fink, G. R. et al., 1991; Wach, A. et al., 1994; Guldener, U. et al., 1996; Goldstein, A. L. et al., 1999).

Durch PCR mit den Primern TRK1-FL-BamHl-Fo, TRK1-FL-Pstl-Re, TRK1-FL-PstI-Fo und TRK1-FL-XhoI-Re für TRK1, bzw. TRK2-DEL-5-Fo-B, TRK2-DEL-5-Re, TRK2-DEL-3-Fo und TRK2-DEL-3-Re für TRK2, sowie TOK1-DEL-5-Fo, TOK1-DEL-5-Re, TOK1-DEL-3-Fo und TOK1-DEL-3-Re für TOK1, wurde je ein etwa 500 bp langes Fragment amplifiziert, das jeweils die Anfangs- bzw. Endregion des Gens darstellt (s. Kapitel 2.3). Die amplifizierten Enden ermöglichen später eine richtige Integration in das Hefegenom. Als DNA-Template diente der Hefestamm *w303 a*/α bzw. *w303 a*/α Δ *trk1.*

### Beispiel 2: Konstruktion der Einfach-, Doppel- und Dreifach-Mutanten

### Beispiel 2 a: Einfach-Knockout

Die konstruierten Deletionskassetten für TRK1, TRK2 und TOK1 wurden jeweils in den diploiden Hefestamm YM 96 (MATa/MATα) transformiert. Durch Wachstum der *trk1-*Mutanten (YM123/124) auf (-)URA/Glc und der *trk2-* (YM158-161) bzw. *tok1*-Mutanten (YM154-157) auf YPD/Geniticin wurde überprüft, ob die Deletionskassetten im Genom integriert waren, da der URA3-Marker in der TRK1-Deletionskassette ein Wachstum auf (-)URA-Medium ermöglicht, der KAN-Marker in der TRK2- bzw. TOK1-Deletionskassette ein Wachstum auf Geneticin (Fink, G. R. et al., 1991).

Durch Replikaplattierung wurden die positiven Kolonien auf eine Sporulationsplatte übertragen, worauf die Sporulation von MATa/MATα diploiden Zellen nach 18-24 h ohne vegetatives Wachstum erfolgt. Nach Zymolasebehandlung und erneutem Wachstum auf YPD wurden dann mit Hilfe des Dissektionsmikroskops von einigen Kolonien Tetraden in je 4 Sporen vereinzelt.

Die Bestimmung des Matingtyps der Sporenkolonien erfolgte durch Paarung mit den entsprechenden Testerstämmen (Fink, G. R. et al., 1991). Die Selektion für Vorhandensein der deletionskassette erfolgte durch replika-Plattierung auf -URA Medium (für *trk1*) bzw. auf Geneticin -enthaltendem Medium für *trk2* und *tok1.* Nach Gewinnung der genomischen DNA der Transformanten durch Hefe-DNA Präparation, wurde das Ergebnis durch diagnostische PCR und Southern Blot bestätigt.

### Beispiel 2 b: Doppel-Knockout

Die TOK1-Deletionskassette wurde in die haploiden Δ*trk1* -Hefestämme YM123 und YM124 transformiert und durch Wachstum auf YPD/Geneticin auf Integration der TOK1-Deletionskassette selektiert. Das Ergebnis wurde mit diagnostischer PCR und Southern Blot überprüft. Von den (+)URA3,(+)KAN (Δ*trk1* Δ*tok1*)-Stämmen wurden Glycerinkulturen angelegt (YM140, YM141, YM143 und YM144).

Einzelkolonien wurden als Patches ausgestrichen, auf 5-FOA replikaplattiert und Kolonien selektioniert, die den URA3-Marker und ein *his*G-Repeat aus der TRK1-Deletionskassette eliminiert hatten (Fink, G. R. et al., 1991). Auf (-)URA/Glc wuchsen demnach keine Kolonien, da das URA3-Gen (in TRK1) zur Uracilsynthese fehlte, auf YPD/Gen dagegen überlebten alle Kolonien, durch das Resistenzgen in der TOK1-Deletionskassette.Um den Kan-Marker aus dem Genom zu entfernen, wurden die (-)URA3-Mutanten mit dem Plasmid pSH47 transformiert, auf dem sich die Gene für Cre-Rekombinase und Uracilsynthese (URA3) befinden. Positive Transformanten wuchsen auf (-)URA/Glc, durch Inkubation in (-)URA/Gal-Flüssigmedium konnte dann die Cre-Rekombinase induziert werden. Dabei wird der Kan-Marker mit einem *loxP-*Repeat eliminiert, zurück bleibt ein *loxP*.

Nach Einstellung der Übernachtkultur auf eine OD₆₀₀ = 5 wurden die Verdünnungen 1:10000 und 1:50000 auf (-)URA/Gal ausplattiert. Patches von Einzelkolonien, auf YPD/Gen replikaplattiert, zeigten kein Wachstum (das heißt der Kan-Marker wurde erfolgreich eliminiert). Um das Plasmid pSH47 wieder zu entfernen, wurde anschließend erneut 2x auf 5-FOA selektioniert. Von den (-)URA(-)KAN (Δ*trk1* Δ*tok1*)-Stämmen wurden Glycerinkulturen angelegt (YM162, YM163 und YM164).

### Beispiel 2 c: Dreifach-Knockout

Von Δ*trk1* Δ*tok1* -Einzelkolonien (YM162 und YM164) wurden Übernachtkulturen in YPD angesetzt, am nächsten Tag mit der *Bsi*WI/*Spe*I verdauten TRK2-Deletionskassette transformiert und auf YPD/KCI/Geneticin ausplattiert. Nach einer Hefe-DNA-Präperation wurde der Dreifach-Knockout durch diagnostische PCR und Southern Blot bestätigt.

**Tabelle 1**

| Reihe oben von links nach rechts: | | | Reihe unten von links nach rechts: | | |
|---|---|---|---|---|---|
| 1. | Marker | | 1. | Marker | |
| 2. | YM 97 | mit TRK1 DiaFo/Re1 | 2. | YM 182 | mit TRK1 DiaFo/Re1 |
| 3. | YM 97 | mit TRK2 DiaFo/Re1 | 3. | YM 182 | mit TRK2 DiaFo/Re1 |
| 4. | YM 97 | mit TOK1 DiaFo/Re1 | 4. | YM 182 | mit TOK1 DiaFo/Re1 |
| 5. | YM 97 | mit TRK1 DiaFo/URARe | 5. | YM 182 | mit TRK1 DiaFo/URARe |
| 6. | YM 97 | mit TRK2 DiaFo/KANRe | 6. | YM 182 | mit TRK2 DiaFo/KANRe |
| 7. | YM 97 | mit TOK1 DiaFo/KANRe | 7. | YM 182 | mit TOK1 DiaFo/KANRe |
| 8. | frei | | 8. | frei | |
| 9. | YM 97 | mit TRK1 DiaFo/Re2 | 9. | YM 182 | mit TRK1 DiaFo/Re2 |
| 10. | YM 97 | mit TRK2 DiaFo/Re2 | 10. | YM 182 | mit TRK2 DiaFo/Re2 |
| 11. | YM 97 | mit TOK1 DiaFo/Re2 | 11. | YM 182 | mit TOK1 DiaFo/Re2 |

### Beispiel 3: Subklonierung und Transformation der humanen Kaliumkanäle in die Doppel- bzw. Dreifachmutante

Die humanen Gene HERG, HCN2, Kv1.5, sowie als Positivkontrollen TRK1 und IRK1 (Meerschweinchen), wurden mittels Restriktionsverdaus aus den Trägerplasmiden (HERG zwischen *Bam*HI in pcDNA; HCN2 zwischen *Nco*I/*Xho*I in pTLN; Kv1.5 zwischen *Nhe*I/*EcoR*I in pcDNA3.1 (-); IRK1 zwischen *Bam*HI/*Eco*RI in pSGEM) ausgeschnitten, durch Gelelektrophorese aufgetrennt und aus dem Gel extrahiert. Jeder humane Kaliumkanal wurde einzeln in den Hefevektor p423-GPD3 (Mumberg, D. et al., 1995; Ronicke, V. et al., 1997) ligiert und in *E.coli* transformiert. Durch Kontrollverdau der Plasmid-Präperationen und Sequenzierung konnte festgestellt werden, welche der Klone das humane Gen integriert hatten. Die Plasmide wurden anschließend in den Δ*trk1* Δ*trk2* Doppel-Knockout (YM 168) und in den Δ*trk1* Δ*trk2* Δ*tok1* Dreifach-Knockout (YM 182) transformiert, und auf (-)HIS/80 mM KCI ausplattiert.

### Beispiel 4: Charakterisierung der Knockout-Stämme

### Beispiel 4 a: Wachstum der Doppel- und Dreifach-Mutanten bei verschiedenen K⁺-Konzentrationen und pH-Werten auf Kulturplatten

Zum Vergleich des unterschiedlichen Kaliumbedarfs der verschiedenen Knockouts wurden die Hefestämme YM 182, YM 168, und YM 97 (WT) auf DPM-Platten mit unterschiedlichen K⁺-Konzentrationen und unterschiedlichen pH-Werten inkubiert. Dazu wurden von den Glycerinkulturen zuerst Patches auf 100 mM KCI/pH 6.5 ausgestrichen. Nach 2 Tagen Wachstum wurde auf 50 mM, 30 mM, und 5 mM KCI replikaplattiert. Dieser Versuch zeigte, daß sowohl der Stamm YM168 (Δ*trk1* Δ*trk2*), als auch der Stamm YM182 *(*Δ*trk1* Δ*trk2* Δ*tok1*) auf 50 mM und 30 mM KCI lebensfähig ist. Zusätzlich zeigte sich, daß der Stamm YM182 in Gegenwart von 30 mM KCI besser wuchs als der Stamm YM168. In Gegenwart von 5 mM KCI waren beide Stämme im Gegensatz zum Wildtyp-Stamm YM97 nicht überlebensfähig.

Zum Test auf pH-Abhängigkeit wurden die drei Stämme zusätzlich auf 100 mM bzw. 5 mM KCI/pH 5.0 und auf 100 mM bzw. 5 mM KCI/pH 4.0 replikaplattiert. Dieser Versuch zeigte, daß sowohl YM168 als auch YM182 bei pH4.0 in Gegenwart von 100 mM KCI und 5 mM KCI nicht überlebensfähig sind. Bei pH5.0 und 100 mM KCI ist der Wachstumsdefekt von YM168 stärker ausgeprägt als bei Stamm YM182.

Der Wachstumsdefekt der Stämme YM168 (Δ*trk1* Δ*trk2*) und YM182 (Δ*trk1* Δ*trk2* Δ*tok1*) wird durch Expression von TRK1 vom Vektor pRS416GAL1 völlig kompensiert.

### Beispiel 4 b: Wachstum von Doppel- und Dreifach-Mutante bei verschiedenen K⁺-Konzentrationen in Flüssigmedium

Zur Charakterisierung der Stämme YM168 (Δ*trk1* Δ*trk2*) und YM182 (Δ*trk1* Δ*trk2* Δ*tok1*), auf denen alle weiteren Versuche basieren, wurde das Wachstumsverhalten der Hefestämme in Flüssigkultur untersucht. Zunächst wurden Über-Nacht-Kulturen in DPM/80 mM KCI angesetzt und am nächsten Morgen mit DPM/5 mM KCI sowie mit DPM/15 mM KCI auf eine OD = 0.05 eingestellt. In definierten Zeitabständen wurde mit Hilfe eines Photometers die Optische Dichte bei 600 nm bestimmt.

Diese Untersuchungen zeigen, daß der Wachstumsdefekt des Stammes YM182 bei 5 mM KCI und bei 15 mM KCI weniger stark ausgeprägt ist als bei Stamm YM168.

### Beispiel 5: Charakterisierung der humanen Kaliumkanäle in Doppel- und Dreifach-Knockout

### Beispiel 5 a: Komplementationsvermögen bei K⁺-Mangel auf Kultur-Platten

Die Stämme YM168 (Δ*trk1* Δ*trk2*) und YM182 (Δ*trk1* Δ*trk2* Δ*tok1*) wurden jeweils mit den humanen Kaliumkanälen Kv1.5 ((Fedida, D. et al., 1998);YM190 bzw. YM195) und HERG1 ((Fedida, D. et al., 1998) ;YM191 bzw. YM196) in p423-GPD3 als Hefeexpression-Vektor transformiert. Als Positivkontrolle dienten gpIRK1 ((Tang, W. et al., 1995);YM193 bzw. YM198) in p423-GPD3 als Hefeexpression-Vektor (Mumberg, D. et al., 1995; Ronicke, V. et al., 1997). Der leere Vektor p423-GPD3 (YM189 bzw. YM194) diente als Negativkontrolle. Die transformierten Hefestämme wurden auf (-)HIS/80 mM KCI-Medium ausplattiert. Danach wurden Patches von Einzelkolonien auf DPM/5 mM KCI (pH 6.5) replikaplattiert, um die Fähigkeit zur Komplementation des Kaliummangels zu überprüfen.

Diese Versuche zeigten, daß die Positivkontrolle gpIRK1 (YM193 bzw. YM198) in p423-GPD3 den Wachstumsdefekt von Doppel- und Dreifach-Knockout vollständig komplementiert. Der leere Vektor p423-GPD3 (YM189 bzw. YM194) als Negativkontrolle ist nicht in der Lage, den Wachstumsdefekt zu komplementieren. Der humane Kaliumkanal Kv1.5 komplementiert den Wachstumsdefekt von Dreifach-Knockout zwar, jedoch signifikant schlechter als die Positivkontrolle gpIRK1. Zudem war zu beobachten, daß der humane Kaliumkanal Kv 1.5 den Doppel-Knockout Δ*trk1* Δ*trk2* nicht komplementiert. Unter den gegebenen Versuchsbedigungen komplementiert der HERG1-Kanal den Wachstumsdefekt von Doppel- und Dreifach-Knockout nicht.

### Beispiel 5 b: Wachstum in Gegenwart von Aktivatoren auf Kultur-Platten

Um den Einfluß von Aktivatoren auf die verschiedenen Kaliumkanäle zu verdeutlichen, wurden die oben angeführten Stämme in Medien inkubiert, die folgende spezifische Aktivatoren enthielten.

Kv1.5: Rb⁺ verlängert die Hyperpolarisationsphase. Dies bedeutet, daß der nach innen gerichtete K⁺-Strom länger anhält und die Möglichkeit zur Komplementation des Wachstumsdefekts erhöht.

HERG: Cs⁺ verlängert die Hyperpolarisationsphase. Dies bedeutet, daß der nach innen gerichtete K⁺-Strom länger anhält und die Möglichkeit zur Komplementation des Wachstumsdefekts erhöht. Dieser kanal wird durch Cs⁺ inhibiert.

IRK1: Cs⁺ blockiert diesen Kanal.

Die Versuche für p423-GPD3-Kv1.5 zeigten, daß der humane Kv1.5 -Kanal in der Gegenwart von 2 mM RbCl in der Lage ist, den Wachstumsdefekt der Δ*trk1* Δ*trk2* Δ*tok1*-Mutante vollständig zu komplementieren (Fig. 3). Die Komplementation des Wachstumsdefekt der Δ*trk1* Δ*trk2* -Mutante ist deutlich schlechter (Fig. 3). Dies stimmt überein mit den unter Beispiel 6 a dargestellten Ergebnissen.

Die Versuche für p423-GPD3-HERG zeigten, daß der humane HERG1 -Kanal in der Gegenwart von 2 mM CsCl in der Lage ist, den Wachstumsdefekt der Δ*trk1* Δ*trk2* Δ*tok1-*Mutante vollständig zu komplementieren (Fig. 4). Die Komplementation des Wachstumsdefekt der Δ*trk1* Δ*trk2* -Mutante ist deutlich schlechter (Fig. 4). Dies stimmt überein mit den unter Beispiel 6a dargestellten Ergebnissen.

### Beispiel 5 c: Komplementation durch den Kv1.5 -Kanals in der Δtrk1 Δtrk2 Δtok1-Mutante in Gegenwart von RbCl in Flüssigmedium

Die Hefestämme YM 194 und YM 195 wurden in DPM/-HIS/5 mM KCI mit 1 mM RbCl auf ihr unterschiedliches Wachstumsverhalten in Flüssigmedium hin überprüft. Hierzu wurden 10 ml Über-Nacht-Kultur in DPM/-HIS/80 mM KCl angesetzt und am nächsten Morgen mit den entsprechenden Medien auf eine OD₆₀₀ = 0.05 eingestellt (20 ml Endvolumen). In definierten Zeitabständen wurde mit Hilfe eines Photometers die Optische Dichte bei 600 nm bestimmt.

Diese Versuche belegen eindeutig, daß die Expression von Kv1.5 vom Vektor p423-GPD3 in einem Hefestamm, der für TRK1, TRK2 und TOK1 deletiert ist, den dadurch entstehenden Wachstumsdefekt komplementieren kann.

In weiteren Versuchen konnte gezeigt werden, daß die Komplementation des Wachstumsdefekts durch Kv1.5, und ebenfalls von gpIRK1, in der Gegenwart von 2 mM CsCl inhibiert wird.

### Beispiel 5 d: Komplementation durch den HERG1 -Kanals in der Δtrk1 Δtrk2 Δtok1-Mutante in Gegenwart von CsCl in Flüssigmedium

Die Hefestämme YM 194 und YM 196 wurden in DPM/-HIS/5 mM KCI mit 1 mM CsCl auf ihr unterschiedliches Wachstumsverhalten in Flüssigmedium hin überprüft. Hierzu wurden 10 ml Über-Nacht-Kultur in DPM/-HIS/80 mM KCI angesetzt und am nächsten Morgen mit den entsprechenden Medien auf eine OD₆₀₀ = 0.05 eingestellt (20 ml Endvolumen). In definierten Zeitabständen wurde mit Hilfe eines Photometers die Optische Dichte bei 600 nm bestimmt. Diese Versuche belegen eindeutig, daß die Expression von HERG1 vom Vektor p423-GPD3 in einem Hefestamm, der für TRK1, TRK2 und TOK1 deletiert ist, den dadurch entstehenden Wachstumsdefekt komplementieren kann.

### Beispiel 6:

Alle Wachstumsassays in der Dreifach-Mutante Δ*trk*1Δ*trk*2Δ*tok*1 wurden in dem Wachstumsmedium DPM (Defined Potassium Medium; bei dem jeweils angegebenen pH-Wert und den jeweils angegebenen Kalium-Konzentrationen durchgeführt.

Als Inhibitoren des humanen HERG1 -K⁺-Kanals wurden die Substanzen Terfenadin (α-(4-Tert-Butylphenyl)-4-(α-hydroxy-αPhenylbenzyl)-1-Piperidinebutanol; HMR), Pimozid (1-(4,4-Bis(P-Fluorophenyl)Butyl)-4-(2-Oxo-1-Benzimidazolinyl)-Piperidine; Sigma, Kat.Nr. P100), Ziprasidon (5-(2-[4-(1,2-Benzisothiazol-3-yl)piperazino]-ethyl)-6-chlor-1,3-dihydro-2H-indol-2-on; HMR), Loratidin (Ethyl-4-(8-chlor-5,6-dihydro-11H-benzo[5,6]cyclohepta[1,2-b]pyridin-11-yliden)-1-piperidincarboxylat; HMR) und Sertindol (1-(2-[4-[5-Chlor-1-(4-Fluorphenyl)-1H-indol-3-yl]-1-piperidinyl]ethyl)-2-imidazolidinon; HMR) eingesetzt (Richelson, E. 1996; Richelson, E. 1999; Delpon, E. et al., 1999; Kobayashi, T. et al., 2000; Drici, M. D. et al., 2000). Zudem wurden mit Diphenhydramin (Sigma, Kat.Nr. D3630) und Fexofenadin (Benzeneacetic acid, 4-[hydroxy-4-[4-(hydroxydiphenylmethyl)-1-piperidinyl]butyl]-α,α-dimethyl, hydrochloride; HMR) (Taglialatela, M. et al., 1999; DuBuske, L. M. 1999) auch Substanzen eingesetzt, die auf Kaliumkanäle nicht hemmend wirken sollten. Alle Substanzen wurden - in DMSO gelöst - zu einer Endkonzentration von 30µM eingesetzt. Zur Kontrolle wurden Zellen mit der gleichen Endkonzentration von 0.5% DMSO ohne Substanz, sowie ohne Zusatz von DMSO oder Substanz gemessen.

Wie in den Abbildungen 1 und 2 beschrieben, ist der humane HERG1-Kanal in der Lage, den Wachstumsdefekt der Dreifachmutante Δ*trk*1Δ*trk*2Δ*tok*1 auf Medium, das lediglich 5mM KCI enthält, zu komplementieren. Es konnte gezeigt werden (Fig. 7, Fig. 8), daß der humane HERG1-Kanal in Gegenwart der Substanzen Terfenadin, Pimozid, Ziprasidon, Sertindol und Loratadin den Wachstumsdefekt der Dreifachmutante Δ*trk*1Δ*trk*2Δ*tok*1 auf Medium, das lediglich 5mM KCI enthält, nicht mehr komplementieren kann.

### Beispiel 7:

Die Inkubation des Wildtypstammes, der alle drei endogenen Kaliumkanalproteine der Hefe exprimiert, mit den Substanzen Terfenadin, Pimozid, Diphenhydramin, Ziprasidon, Loratidin, Fexofenadin und Sertindol zeigte, daß es sich bei Terfenadin, Loratadin und Sertindol um spezifische Inhibitoren des humanen HERG1-Kanals handelt (Figur 9).

Den vorliegenden Ergebnissen zufolge sind Pimozid und Ziprasidon als eher unspezifische Inhibitoren einzuordnen. Das heißt, diese Substanzen hemmen möglicherweise nicht nur den humanen HERG1 -Kanal, sondern auch die endogenen Kaliumkanäle der Hefe Saccharomyces cerevisiae. Die vorliegenden Ergebnisse konnten jedoch nicht ausschließen, daß der inhibitorische Effekt, der für diese Substanzen gefunden wurde, möglicherweise auch auf eine Hemmung anderer Proteine zurückzuführen ist, die für das Wachstum von Hefezellen essentiell sind. Um diese Möglichkeit zu untersuchen, wurde die Wirkung dieser Substanzen auch in einem Wachstums-Medium getestet, das 80mM KCI enthält.

Diese Untersuchungen zeigten (Figur 10), daß Pimozid unspezifisch die Aktivität der essentiellen endogenen Kaliumkanäle TRK1 und TRK2 hemmt. Das Fehlen eines inhibitorischen Effekts bei höheren Kaliumkonzentrationen läßt darauf schließen, daß Pimozid keinen generell toxischen Effekt auf Hefezellen besitzt. Im Gegensatz dazu konnte gezeigt werden, daß Ziprasidon das Wachstum der Hefezellen auch bei höheren Kaliumkonzentrationen hemmt und somit einen toxischen Effekt auf *Saccharomyces cerevisiae* hat. Es bleibt zu ermitteln, welches mögliche Zielprotein in der Hefe für diesen Effekt verantwortlich sein könnte.

Zusammengefaßt zegen diese Experimente, daß es mit dem beschriebenen System praktisch möglich ist, in der Hefe *Saccharomyces cerevisiae* Substanzen zu identifizieren, die menschliche Kaliumkanäle spezifisch inhibieren.

Ergebnisse siehe Figur 10.

### Beispiel 8:

Die humanen Kaliumkanäle HERG1 und Kv1.5 komplementieren den Wachstumsdefekt der Doppelmutante Δ*trk*1Δ*trk*2 nicht (Figur 11 und Figur 12).
Ergebnisse: Figur 11 und 12.

Die Figuren 11 und 12 zeigen , daß die humanen Kaliumkanäle HERG1 und Kv1.5 den Wachstumsdefekt der Doppelmutante Δ*trk*1Δ*trk*2 nicht komplementieren (jeweils 4. Ausschnitt der Figur 11 und 12). Der Vergleich zur Negativkontrolle, d.h. dem Leervektor p423GPD in der Dreifachmutante Δ*trk*1Δ*trk*2Δ*trk*1 (jeweils 1. Ausschnitt der Figur 11 und 12) zeigt kein verbessertes Wachstum. Die Negativkontrolle p423GPD in der Doppelmutante Δ*trk*1Δ*trk*2 ist nicht dargestellt, unterscheidet sich jedoch nicht von der Negativkontrolle p423GPD in der Dreifachmutante Δ*trk*1Δ*trk*2Δ*trk*1 Im Gegensatz dazu komplementieren die humanen Kaliumkanäle HERG1 und Kv1.5 den Wachstumsdefekt der Dreifachmutante Δ*trk*1Δ*trk*2Δ*trk*1 (jeweils 3. Ausschnitt der Figur 11 und 12).

### Beispiel 9:

Der humane Kaliumkanal ROMK2 ((Shuck, M. E. et al., 1994; Bock, J. H. et al., 1997); Sequenz SEQ ID NO. 31 hROMK2) wurde in den Hefevektor p423GPD subkloniert und in die Dreifach-Mutante Δ*trk*1Δ*trk*2Δ*trk*1 transformiert. Die Untersuchungen zeigten, daß auch dieser humane Kaliumkanal in der Lage ist, den Wachstumsdefekt der Dreifach-Mutante Δ*trk*1Δ*trk*2Δ*trk*1 zu komplementieren.

Bislang wurde noch nicht untersucht, ob dieser humane Kaliumkanal den Wachstumsdefekt der Doppelmutante Δ*trk*1Δ*trk*2 komplementieren kann. Bislang sind noch keine Substanzen bekannt, die den ROMK2 -Kanal spezifisch inhibieren.

### Ergebnisse siehe Figur 13.

### Literatur

Curran, M. E., Landes, G. M., and Keating, M. T. Molecular cloning, characterization, and genomic localization of a human potassium channel gene. Genomics 12: 729 - 737. (1992)
Dascal, N., Schreibmayer, W., Lim, N. F., Wang, W., Chavkin, C., DiMagno, L., Labarca, C., Kieffer, B. L., Gaveriaux-Ruff, C., and Trollinger, D. Atrial G protein-activated K+ channel: expression cloning and molecular properties. Proc.Natl.Acad.Sci. U.S.A. 90: 10235 - 10239. (1993)
Fairman, C., Zhou, X., and Kung, C. Potassium uptake through the TOK1 K+ channel in the budding yeast. J.Membr.Biol. 168: 149 - 157. (1999)
Fedida, D., Chen, F. S., and Zhang, X. The 1997 Stevenson Award Lecture. Cardiac K+ channel gating: cloned delayed rectifier mechanisms and drug modulation. Can.J.Physiol.Pharmacol. 76: 77 - 89. (1998)
Fink, G. R. and Guthrie, C. Guide to Yeast Genetics and Molecular Biology. Guthrie, C. and Fink, G. R. (194). 1991. Academic Presss, Inc. Methods in Enzymology. Ref Type: Book, Whole
Gaber, R. F., Styles, C. A., and Fink, G. R. TRK1 encodes a plasma membrane protein required for high-affinity potassium transport in Saccharomyces cerevisiae. Mol.Cell Biol. 8: 2848 - 2859. (1988)
Goldstein, A. L. and McCusker, J. H. Three new dominant drug resistance cassettes for gene disruption in Saccharomyces cerevisiae [In Process Citation] Yeast. 15: 1541 - 1553. (1999)
Goldstein, S. A., Price, L. A., Rosenthal, D. N., and Pausch, M. H. ORK1, a potassiumselective leak channel with two pore domains cloned from Drosophila melanogaster by expression in Saccharomyces cerevisiae [published erratum appears in Proc Natl Acad Sci U S A 1999 Jan 5,96(1):318] Proc.Natl.Acad.Sci.U.S.A. 93: 13256 - 13261. (1996)
Guldener, U., Heck, S., Fielder, T., Beinhauer, J., and Hegemann, J. H. A new efficient gene disruption cassette for repeated use in budding yeast. Nucleic.Acids.Res. 24: 2519 - 2524. (1996)
Ikeda, K., Kobayashi, K., Kobayashi, T., Ichikawa, T., Kumanishi, T., Kishida, H., Yano, R., and Manabe, T. Functional coupling of the nociceptin/orphanin FQ receptor with the G- protein-activated K+ (GIRK) channel. Brain Res.Mol.Brain Res. 45: 117 - 126. (1997)
Itoh, T., Tanaka, T., Nagai, R., Kamiya, T., Sawayama, T., Nakayama, T., Tomoike, H., Sakurada, H., Yazaki, Y., and Nakamura, Y. Genomic organization and mutational analysis of HERG, a gene responsible for familial long QT syndrome. Hum.Genet. 102: 435 - 439. (1998)
Jan, L. Y. and Jan, Y. N. Cloned potassium channels from eukaryotes and prokaryotes. Annu.Rev.Neurosci. 20:91-123: 91 - 123. (1997)
Jelacic, T. M., Sims, S. M., and Clapham, D. E. Functional expression and characterization of G-protein-gated inwardly rectifying K+ channels containing GIRK3. J.Membr.Biol. 169: 123 - 129. (1999)
Ketchum, K. A., Joiner, W. J., Sellers, A. J., Kaczmarek, L. K., and Goldstein, S. A. A new family of outwardly rectifying potassium channel proteins with two pore domains in tandem. Nature 376: 690 - 695. (1995)
Ko, C. H., Buckley, A. M., and Gaber, R. F. TRK2 is required for low affinity K+ transport in Saccharomyces cerevisiae. Genetics 125: 305 - 312. (1990)
Ko, C. H. and Gaber, R. F. TRK1 and TRK2 encode structurally related K+ transporters in Saccharomyces cerevisiae. Mol.Cell Biol. 11: 4266 - 4273. (1991)
Kubo, Y., Reuveny, E., Slesinger, P. A., Jan, Y. N., and Jan, L. Y. Primary structure and functional expression of a rat G-protein-coupled muscarinic potassium channel [see comments] Nature 364: 802 - 806. (1993)
Ludwig, A., Zong, X., Stieber, J., Hullin, R., Hofmann, F., and Biel, M. Two pacemaker channels from human heart with profoundly different activation kinetics. EMBO J. 18: 2323 - 2329. (1999)
Madrid, R., Gomez, M. J., Ramos, J., and Rodriguez-Navarro, A. Ectopic potassium uptake in trk1 trk2 mutants of Saccharomyces cerevisiae correlates with a highly hyperpolarized membrane potential. J.Biol.Chem. 273: 14838 - 14844. (1998)
Main, M. J., Brown, J., Brown, S., Fraser, N. J., and Foord, S. M. The CGRP receptor can couple via pertussis toxin sensitive and insensitive G proteins. FEBS Lett. 441: 6 - 10. (1998)
Mumberg, D., Muller, R., and Funk, M. Yeast vectors for the controlled expression of heterologous proteins in different genetic backgrounds. Gene 156: 119 - 122. (1995)
Myers, A. M., Pape, L. K., and Tzagoloff, A. Mitochondrial protein synthesis is required for maintenance of intact mitochondrial genomes in Saccharomyces cerevisiae. EMBO J. 4: 2087 - 2092. (1985)
Nakamura, R. L., Anderson, J. A., and Gaber, R. F. Determination of key structural requirements of a K+ channel pore. J.Biol.Chem. 272: 1011 - 1018. (1997)
Roberds, S. L. and Tamkun, M. M. Cloning and tissue-specific expression of five voltage-gated potassium channel cDNAs expressed in rat heart. Proc.Natl.Acad.Sci.U.S.A. 88: 1798 - 1802. (1991)
Ronicke, V., Graulich, W., Mumberg, D., Muller, R., and Funk, M. Use of conditional promoters for expression of heterologous proteins in Saccharomyces cerevisiae. Methods Enzymol. 283:313-22: 313 - 322. (1997)
Sanguinetti, M. C. and Zou, A. Molecular physiology of cardiac delayed rectifier K+ channels. Heart Vessels Suppl 12: 170 - 172. (1997)
Schreibmayer, W., Dessauer, C. W., Vorobiov, D., Gilman, A. G., Lester, H. A., Davidson, N., and Dascal, N. Inhibition of an inwardly rectifying K+ channel by G-protein alpha- subunits. Nature 380: 624 - 627. (1996)
Smith, F. W., Ealing, P. M., Hawkesford, M. J., and Clarkson, D. T. Plant members of a family of sulfate transporters reveal functional subtypes. Proc.Natl.Acad.Sci.U.S.A. 92: 9373 - 9377. (1995)
Snyders, D. J., Tamkun, M. M., and Bennett, P. B. A rapidly activating and slowly inactivating potassium channel cloned from human heart. Functional analysis after stable mammalian cell culture expression. J.Gen.Physiol. 101: 513 - 543. (1993)
Taglialatela, M., Castaldo, P., Pannaccione, A., Giorgio, G., and Annunziato, L. Human ether-a-gogo related gene (HERG) K+ channels as pharmacological targets: present and future implications. Biochem.Pharmacol. 55: 1741 - 1746. (1998)
Tang, W., Ruknudin, A., Yang, W. P., Shaw, S. Y., Knickerbocker, A., and Kurtz, S. Functional expression of a vertebrate inwardly rectifying K+ channel in yeast. Mol.Biol.Cell 6: 1231 - 1240. (1995)
Wach, A., Brachat, A., Pohlmann, R., and Philippsen, P. New heterologous modules for classical or PCR-based gene disruptions in Saccharomyces cerevisiae. Yeast. 10: 1793 - 1808. (1994)
Wang, Q., Chen, Q., and Towbin, J. A. Genetics, molecular mechanisms and management of long QT syndrome. Ann.Med. 30: 58 - 65. (1998)
Wilde, A. A. and Veldkamp, M. W. Ion channels, the QT interval, and arrhythmias. Pacing.Clin.Electrophysiol. 20: 2048 - 2051. (1997)
Wischmeyer, E., Doring, F., Spauschus, A., Thomzig, A., Veh, R., and Karschin, A. Subunit interactions in the assembly of neuronal Kir3.0 inwardly rectifying K+ channels. Mol.Cell Neurosci. 9: 194 - 206. (1997)
Yamada, M., Inanobe, A., and Kurachi, Y. G protein regulation of potassium ion channels. Pharmacol.Rev. 50: 723 - 760. (1998)
Bock, J. H., Shuck, M. E., Benjamin, C. W., Chee, M., Bienkowski, M. J., and Slightom, J. L. Nucleotide sequence analysis of the human KCNJ1 potassium channel locus Gene 188: 9 - 16. (1997)
Delpon, E., Valenzuela, C., and Tamargo, J. Blockade of cardiac potassium and other channels by antihistamines Drug Saf 21 Suppl 1:11-8; discussion 81-7: 11 - 18. (1999)
Drici, M. D. and Barhanin, J. Cardiac K+ channels and drug-acquired long QT syndrome Therapie 55: 185 - 193. (2000)
DuBuske, L. M. Second-generation antihistamines: the risk of ventricular arrhythmias Clin. Ther. 21: 281 - 295. (1999)
Itoh, T., Tanaka, T., Nagai, R., Kikuchi, K., Ogawa, S., Okada, S., Yamagata, S., Yano, K., Yazaki, Y., and Nakamura, Y. Genomic organization and mutational analysis of KVLQT1, a gene responsible for familial long QT syndrome Hum.Genet. 103: 290 - 294. (1998)
Kobayashi, T., Ikeda, K., and Kumanishi, T. Inhibition by various antipsychotic drugs of the G-protein-activated inwardly rectifying K(+) (GIRK) channels expressed in xenopus oocytes Br.J.Pharmacol. 129: 1716 - 1722. (2000)
Richelson, E. Preclinical pharmacology of neuroleptics: focus on new generation compounds J.Clin.Psychiatry 57 Suppl 11:4-11: 4 - 11. (1996)
Richelson, E. Receptor pharmacology of neuroleptics: relation to clinical effects [see comments] J.Clin.Psychiatry 60 Suppl 10:5-14: 5 - 14. (1999)
Shuck, M. E., Bock, J. H., Benjamin, C. W., Tsai, T. D., Lee, K. S., Slightom, J. L., and Bienkowski, M. J. Cloning and characterization of multiple forms of the human kidney ROM-K potassium channel J.Biol.Chem. 269: 24261 - 24270. (1994)
Taglialatela, M., Castaldo, P., Pannaccione, A., Giorgio, G., Genovese, A., Marone, G., and Annunziato, L. Cardiac ion channels and antihistamines: possible mechanisms of cardiotoxicity Clin.Exp.Allergy 29 Suppl 3:182-9: 182 - 189. (1999)

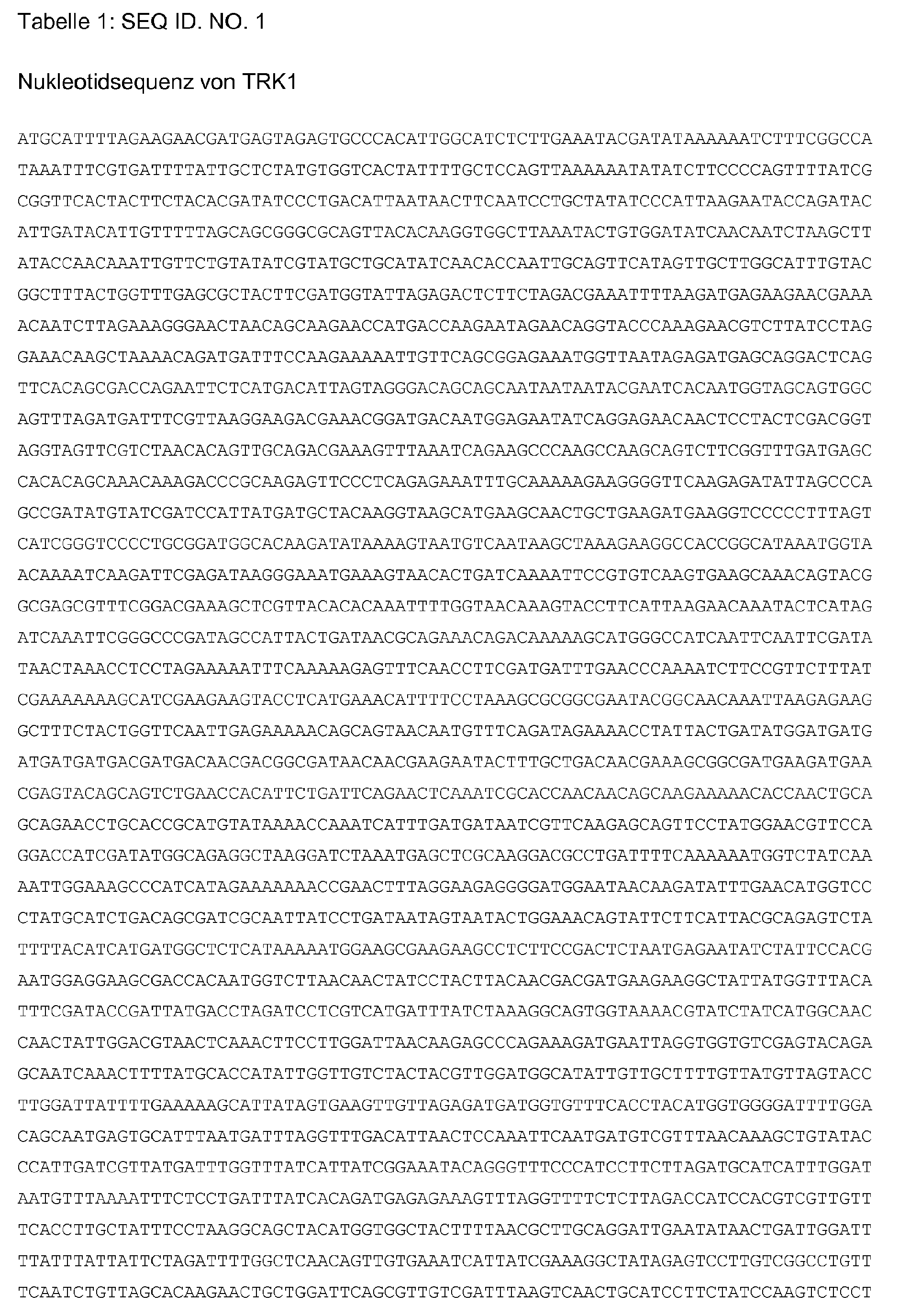

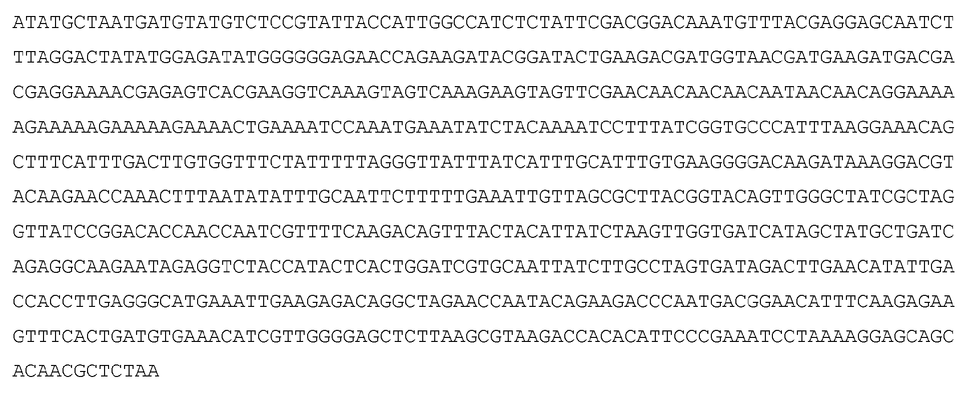

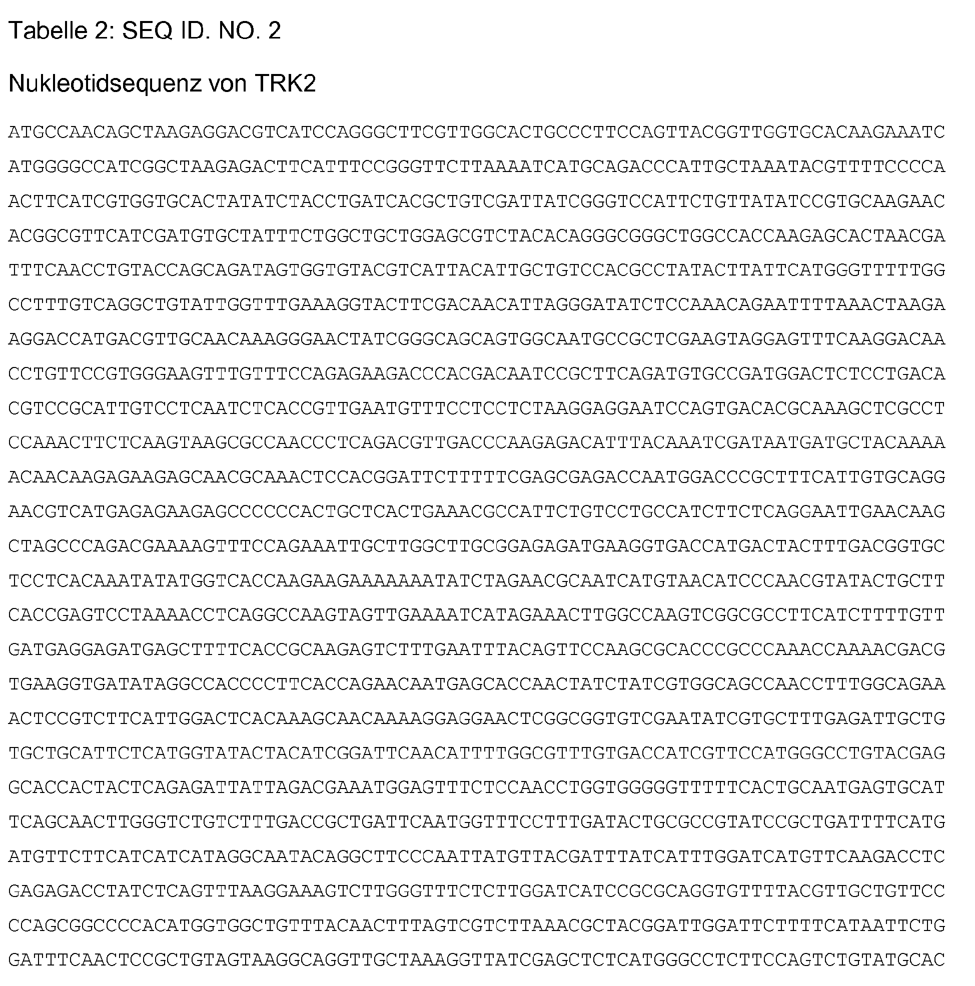

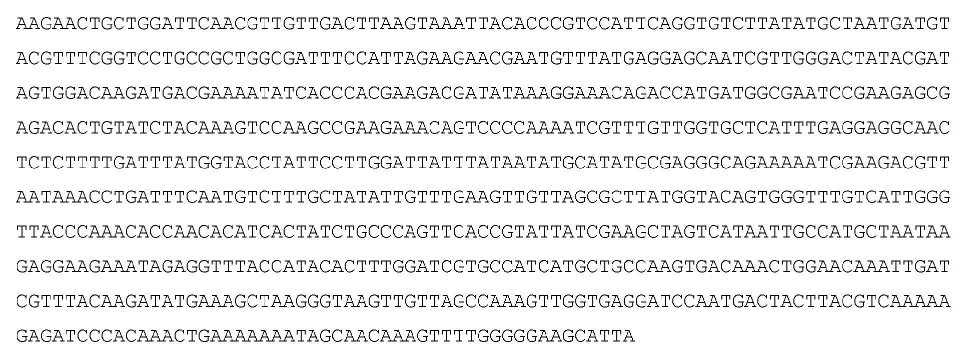

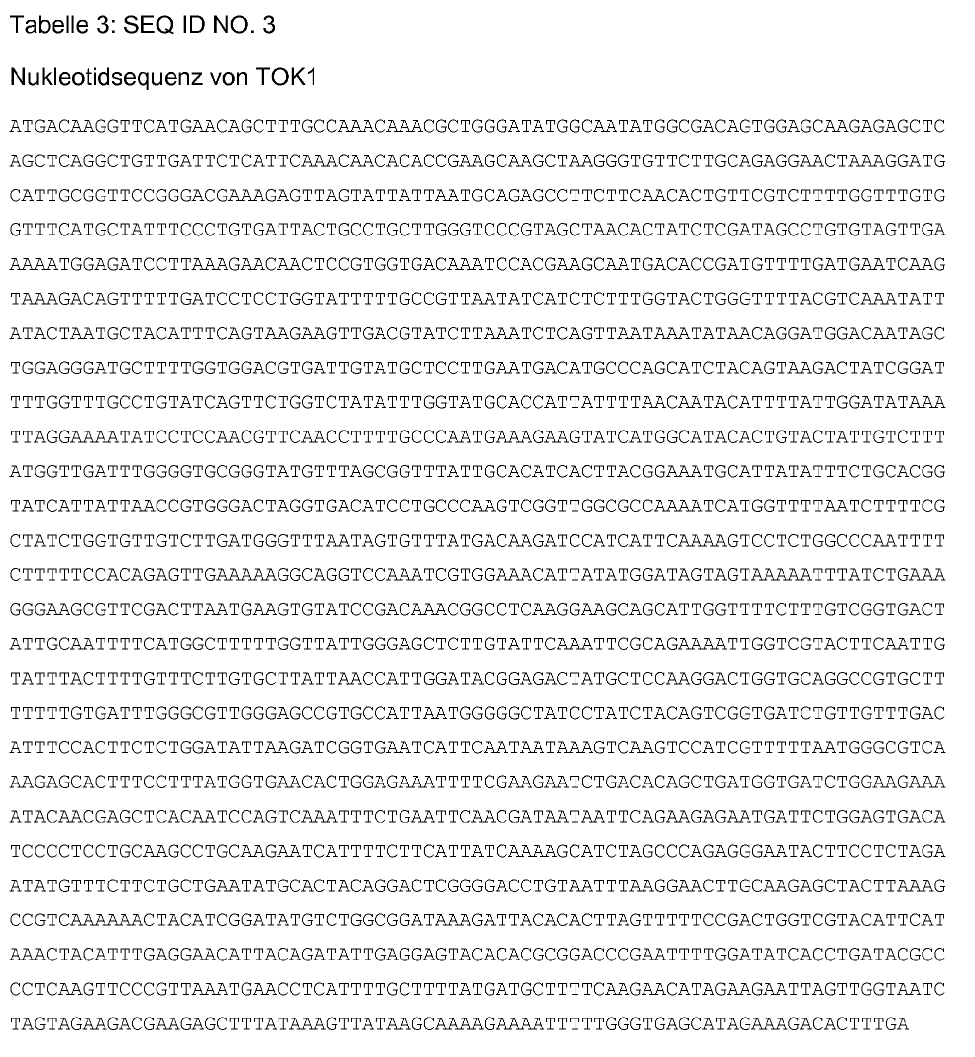

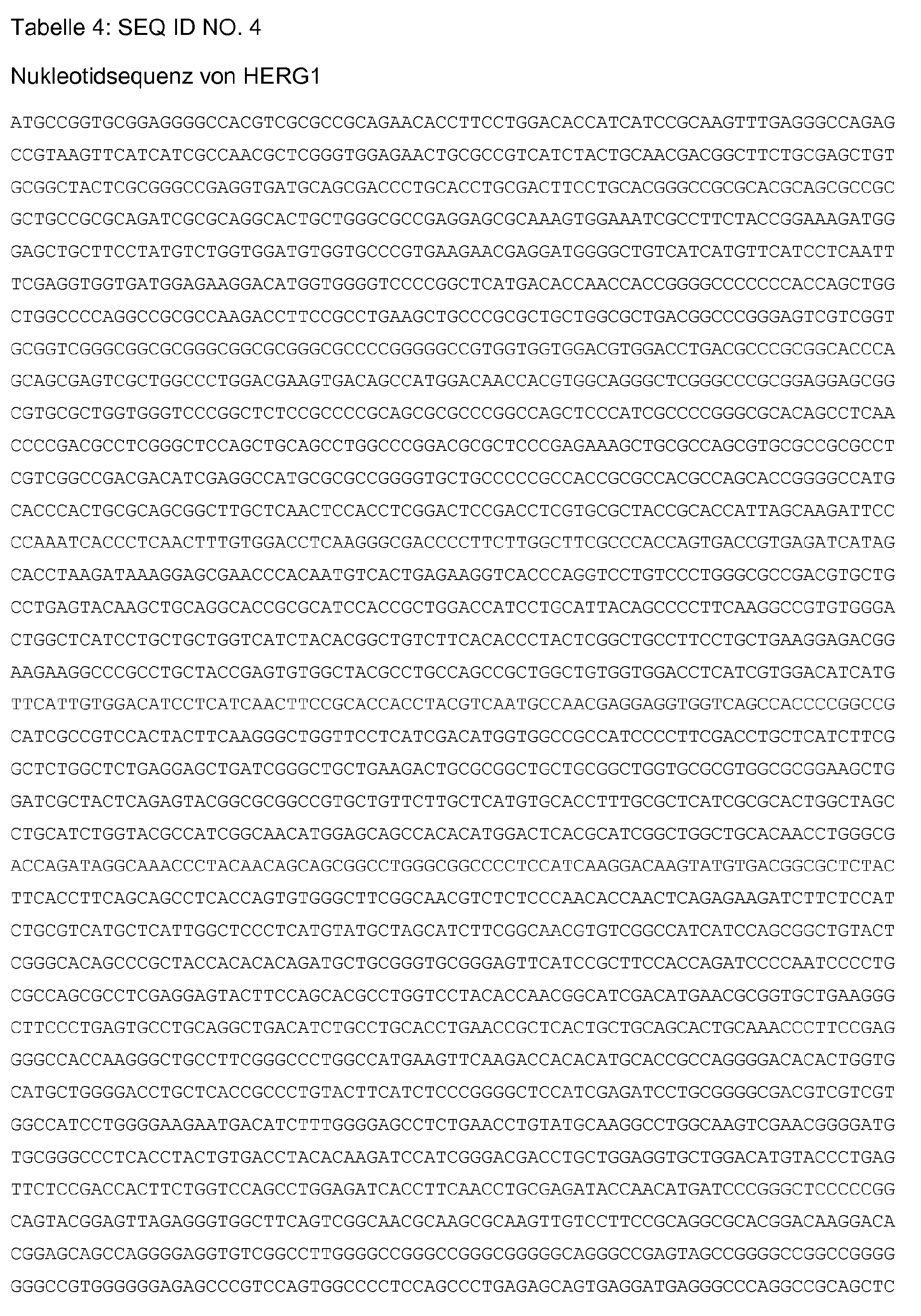

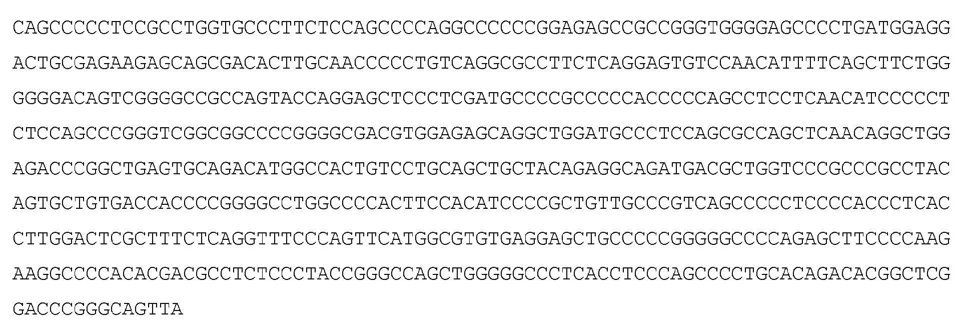

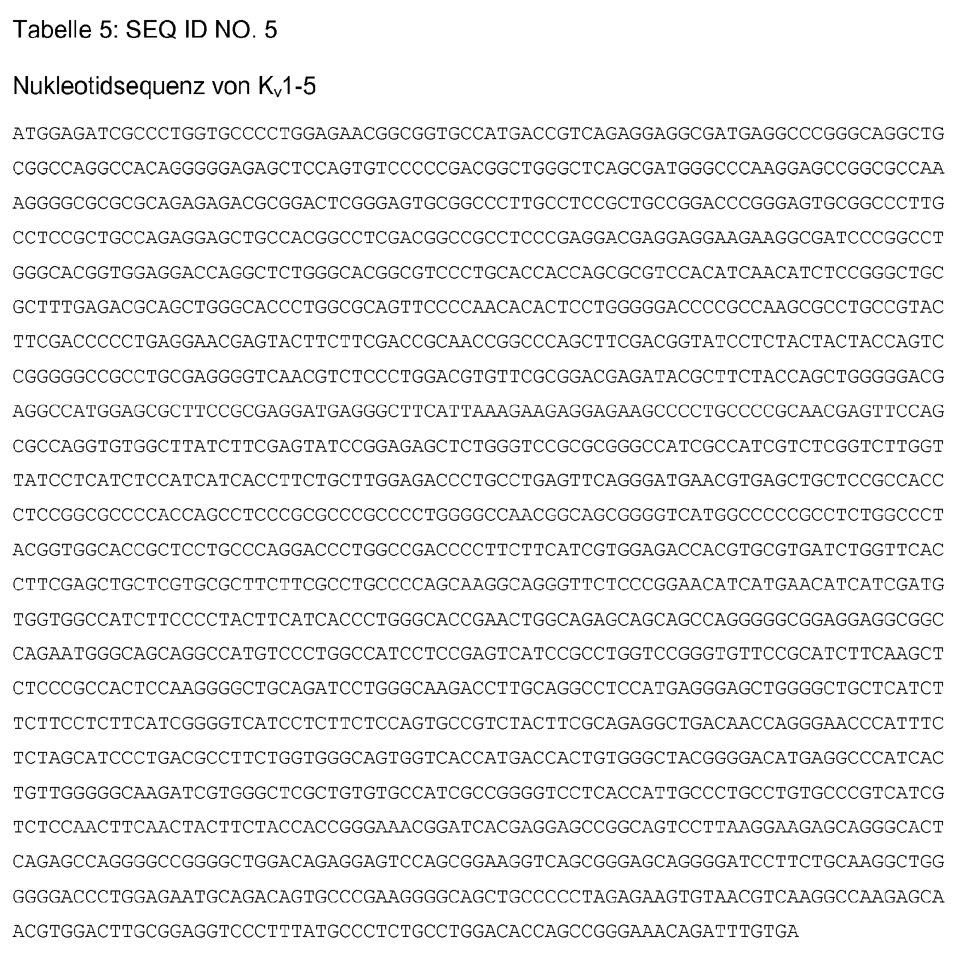

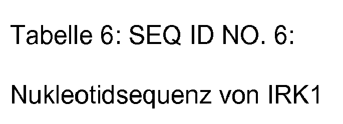

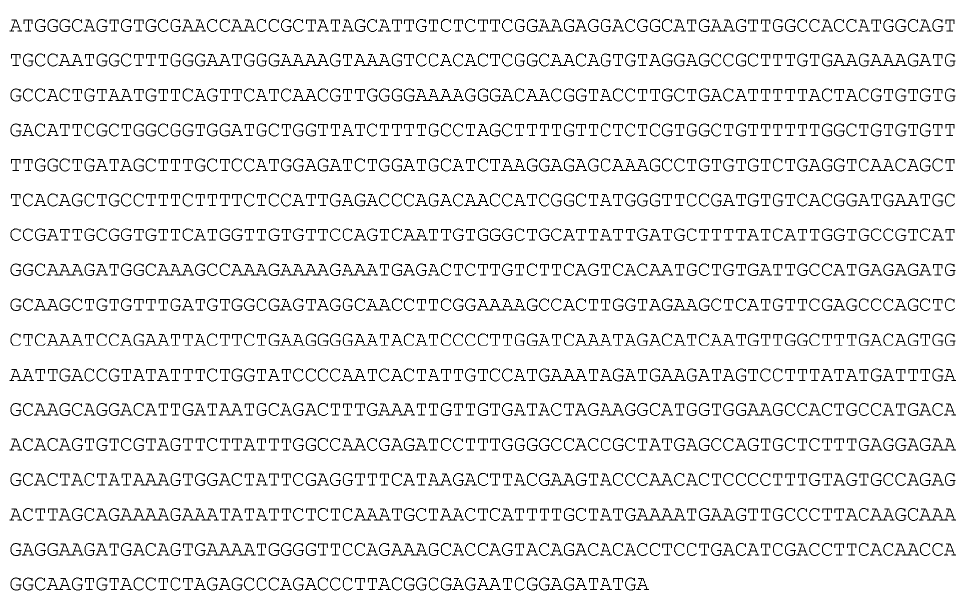

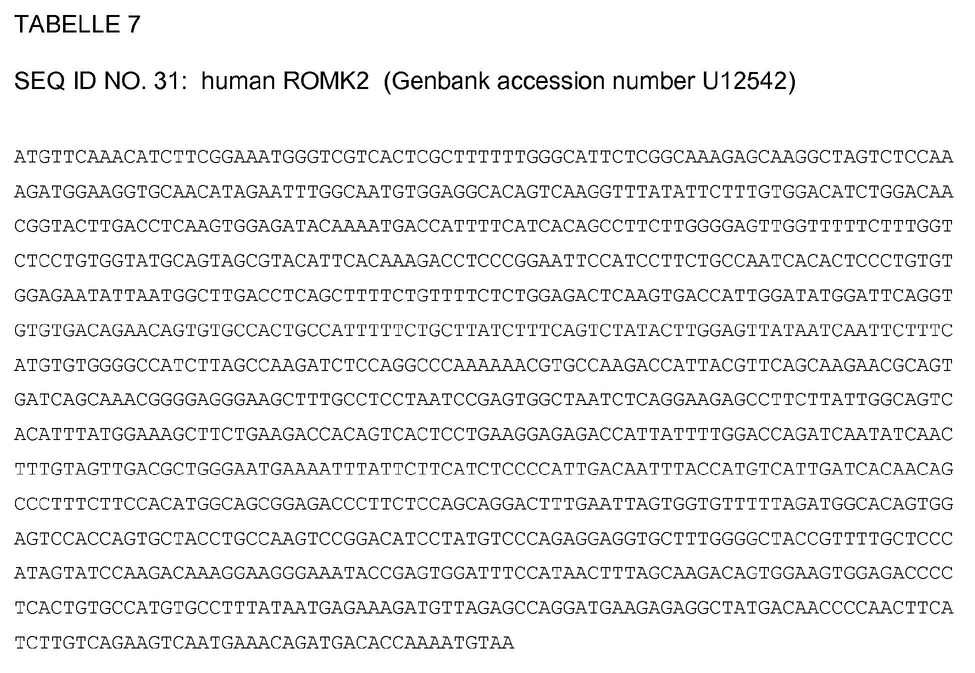

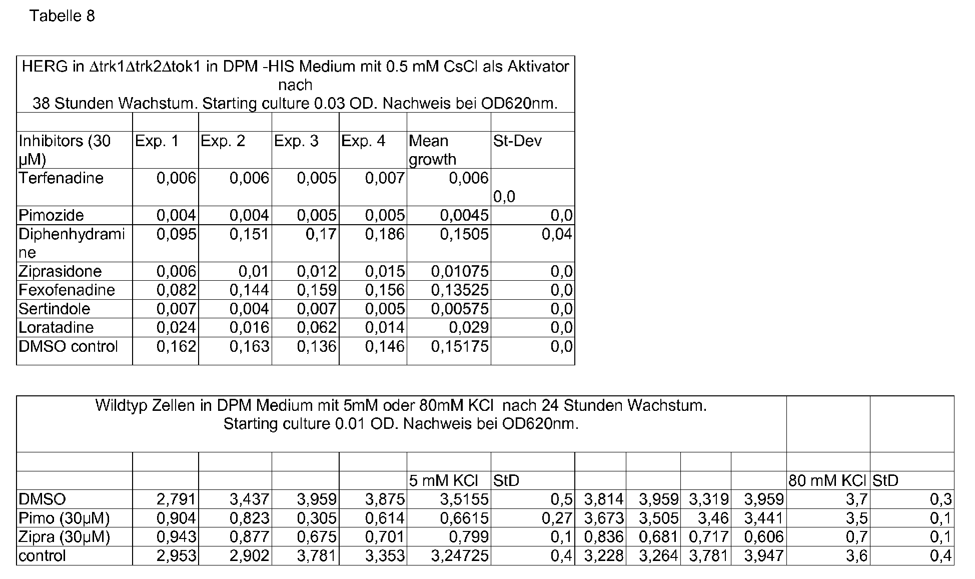

**TABELLE 9**

| LacZ in Wildtypzellen in DPM -HIS/-TRP Medium mit 0.5 mM CsCl als Aktivator nach 24 Stunden Wachdstum; Nachweis mit TROPIX-kit. 1. ASSAY | | | | | | |
|---|---|---|---|---|---|---|
| | | | | | | |

| Inhibitors (30 µM) | Exp. 1 | Exp. 2 | Exp. 3 | Exp. 4 | Mean growth | St-Dev |
|---|---|---|---|---|---|---|
| Terfenadine | 4497 | 4481 | 5058 | 5381 | 4854,25 | 441,936176 |
| Pimozide | 357,4 | 747,9 | 804,6 | 585,4 | 623,825 | 200,443648 |
| Diphenhydramine | 2806 | 3181 | 4178 | 4864 | 3757,25 | 937,881789 |
| Ziprasidone | 55,32 | 70,29 | 70,3 | 77,18 | 68,2725 | 9,22481933 |
| Fexofenadine | 3326 | 2938 | 3377 | 4659 | 3575 | 748,783458 |
| Sertindole | 4165 | 2099 | 4588 | 3069 | 3480,25 | 1121,45304 |
| Loratadine | 4905 | 5141 | 1857 | 3266 | 3792,25 | 1536,17173 |
| DMSO control | 3172 | 4129 | 4984 | 5077 | 4340,5 | 888,190858 |
| | | | | | | |
| | | | | | | |

| LacZ in Wildtypzellen in DPM -HIS/-TRP Medium mit 0.5 mM CsCl als Aktivator nach 24 Stunden Wachstum; Nachweis mit TROPIX-kit. 2. ASSAY | | | | | | |
|---|---|---|---|---|---|---|
| | | | | | | |

| Inhibitors (30 µM) | Exp. 1 | Exp. 2 | Exp. 3 | Exp. 4 | Mean g rowth | St-Dev |
|---|---|---|---|---|---|---|
| Terfenadine (0.5 Cs) | 3439 | 3795 | 3698 | 3388 | 3580 | 197,394698 |
| Pimozide (0.5 Cs) | 905 | 2176 | 496,5 | 573,4 | 1037,725 | 779,2749 |
| Diphenhydramine (0.5 Cs) | 3468 | 2980 | 3062 | 3561 | 3267,75 | 289,383684 |
| Ziprasidone (0.5 Cs) | 62,52 | 44,3 | 49,71 | 51,87 | 52,1 | 7,64158361 |
| Fexofenadine (0.5 Cs) | 3533 | 3502 | 3661 | 3569 | 3566,25 | 68,8446318 |
| Sertindole (0.5 Cs) | 3992 | 3076 | 3972 | 2782 | 3455,5 | 619,738386 |
| Loratadine (0.5 Cs) | 3553 | 1965 | 3590 | 2478 | 2896,5 | 807,211042 |
| DMSO control (0.5 Cs) | 3520 | 3218 | 3460 | 3087 | 3321,25 | 203,540946 |

**Tabelle 10**

| HERG in Δtrk1Δtrk2Δtok1 in DPM -HIS Medium mit 0.5 mM CsCl als Aktivator nach 38 Stunden Wachstum. Starting culture 0.03 OD. Nachweis mit OD620nm. | | | | | | |
|---|---|---|---|---|---|---|
| | | | | | | |

| Inhibitors (30 µM) | Exp. 1 | Exp. 2 | Exp. 3 | Exp. 4 | Mean growth | St-Dev |
|---|---|---|---|---|---|---|
| Terfenadine | 0,006 | 0,006 | 0,005 | 0,007 | 0,006 | 0,0008165 |
| Pimozide | 0,004 | 0,004 | 0,005 | 0,005 | 0,0045 | 0,0005773 |
| | | | | | | 5 |
| Diphenhydramine | 0,095 | 0,151 | 0,17 | 0,186 | 0,1505 | 0,0396694 |
| Ziprasidone | 0,006 | 0,01 | 0,012 | 0,015 | 0,01075 | 0,0037749 |
| | | | | | | 2 |
| Fexofenadine | 0,082 | 0,144 | 0,159 | 0,156 | 0,13525 | 0,0360867 |
| Sertindole | 0,007 | 0,004 | 0,007 | 0,005 | 0,00575 | 0,0015 |
| Loratadine | 0,024 | 0,016 | 0,062 | 0,014 | 0,029 | 0,0224202 |
| | | | | | | 3 |
| DMSO control | 0,162 | 0,163 | 0,136 | 0,146 | 0,15175 | 0,0130735 |
| | | | | | | 1 |

**TABELLE 11**

| Wildtypzellen in DPM Medium mit 5mM or 80mM KCI nach 24 Stunden Wachstum. Starting culture 0.01 OD. Nachweis bei OD620nm. | | | | | | | | | | | | |
|---|---|---|---|---|---|---|---|---|---|---|---|---|
| | | | | | 5 mM KCI | StD | | | | | 80 mM KCI | StD |
| DMSO | 2,791 | 3,437 | 3,959 | 3,875 | 3,5155 | 0,5344763 | 3,814 | 3,959 | 3,319 | 3,959 | 3,76275 | 0,3036273 |
| | | | | | | 8 | | | | | | 8 |
| Pimo (30µM) | 0,904 | 0,823 | 0,305 | 0,614 | 0,6615 | 0,2672308 | 3,673 | 3,505 | 3,46 | 3,441 | 3,51975 | 0,1056326 |
| | | | | | | 6 | | | | | | 2 |
| Zipra (30µM) | 0,943 | 0,877 | 0,675 | 0,701 | 0,799 | 0,1314026 | 0,836 | 0,681 | 0,717 | 0,606 | 0,71 | 0,0958853 |
| | | | | | | 9 | | | | | | 5 |
| control | 2,953 | 2,902 | 3,781 | 3,353 | 3,24725 | 0,4090039 | 3,228 | 3,264 | 3,781 | 3,947 | 3,555 | 0,3634785 |
| | | | | | | 7 | | | | | | 6 |

**TABELLE 12**

| p423GPD (YM194) und p423GPD-ROMK2 (YM256) in Dtrk1 Dtrk2Dtok1 in DPM -HIS 5mM KCI, pH 6.5 nach 24 Stunden Wachstum; starting OD 0.01. Durchschnittswerte | | | | |
|---|---|---|---|---|
| | 194 | SD | 256 | SD |
| DMSO | 0,023 | 0,0036 | 0,19 | 0,013 |
| (0.5%) | | | | |
| Cells | 0,028 | 0,0012 | 0,23 | 0,011 |
| 2 mM RbCl | 0,048 | 0,0052 | 0,44 | 0,033 |
| Signal to noise ratio | | | | |
| | S/N | | | |
| DMSO | 8,2 | | | |
| (0.5%) | | | | |
| Cells | 8,3 | | | |
| 2 mM RbCl | 8,46 | | | |

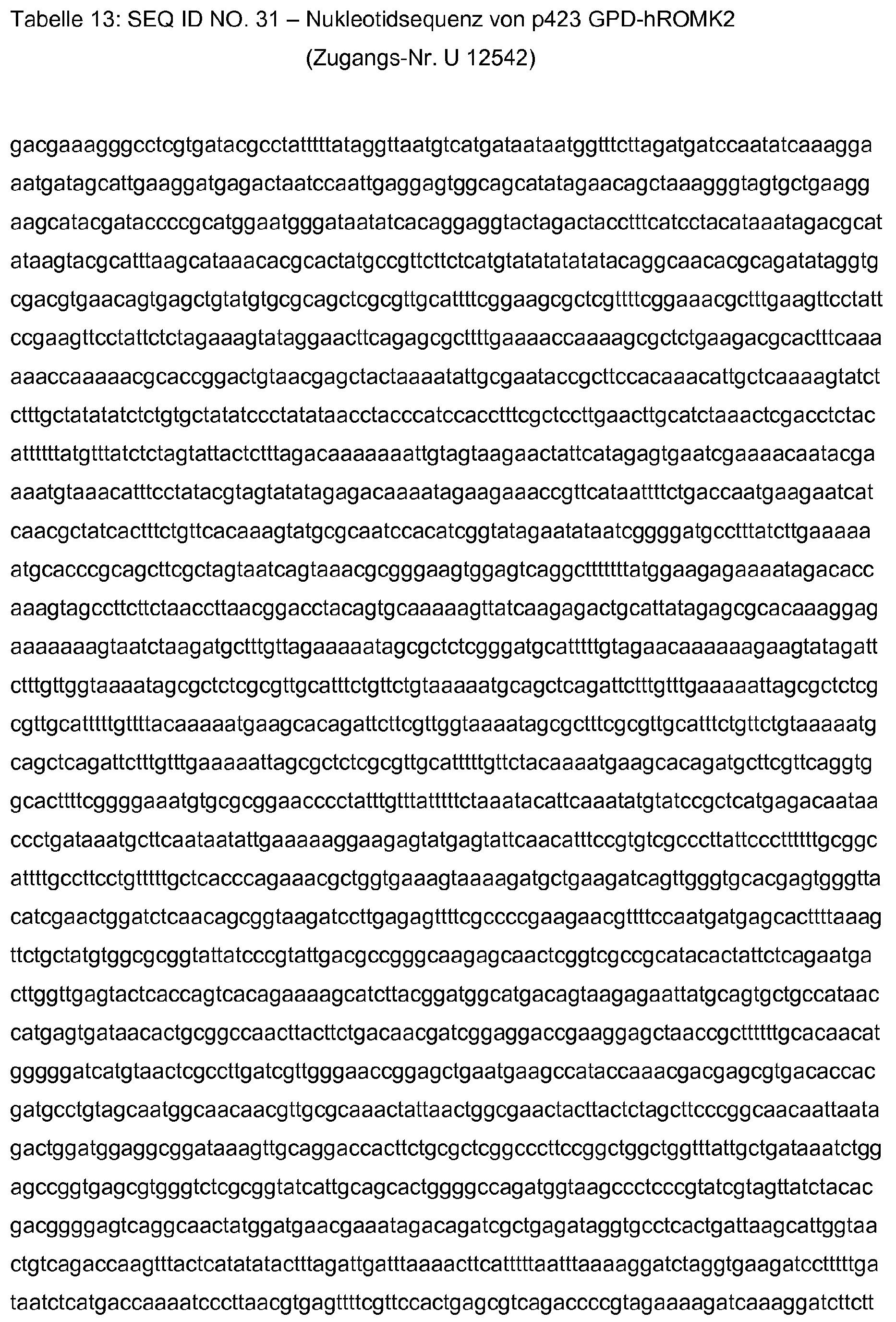

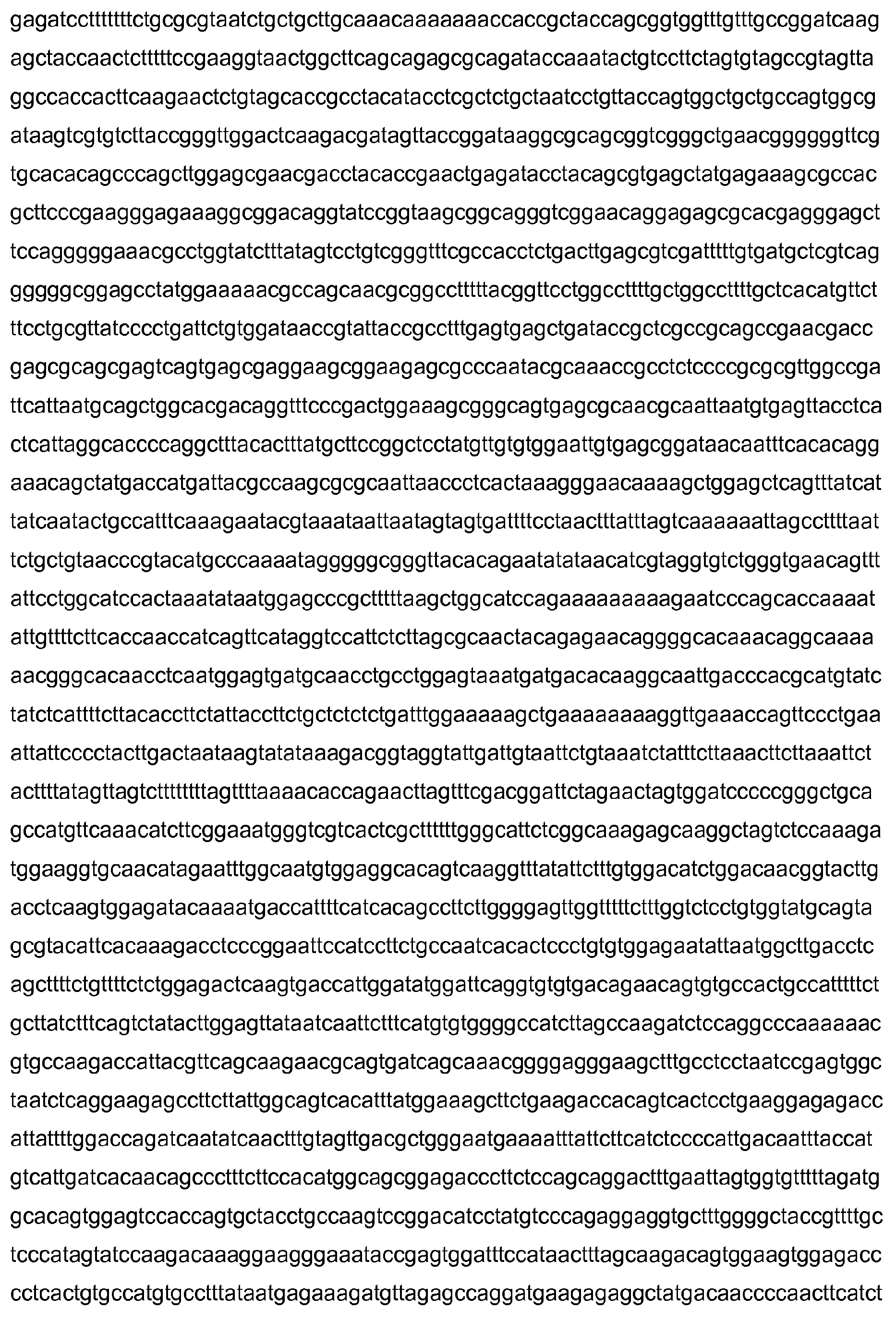

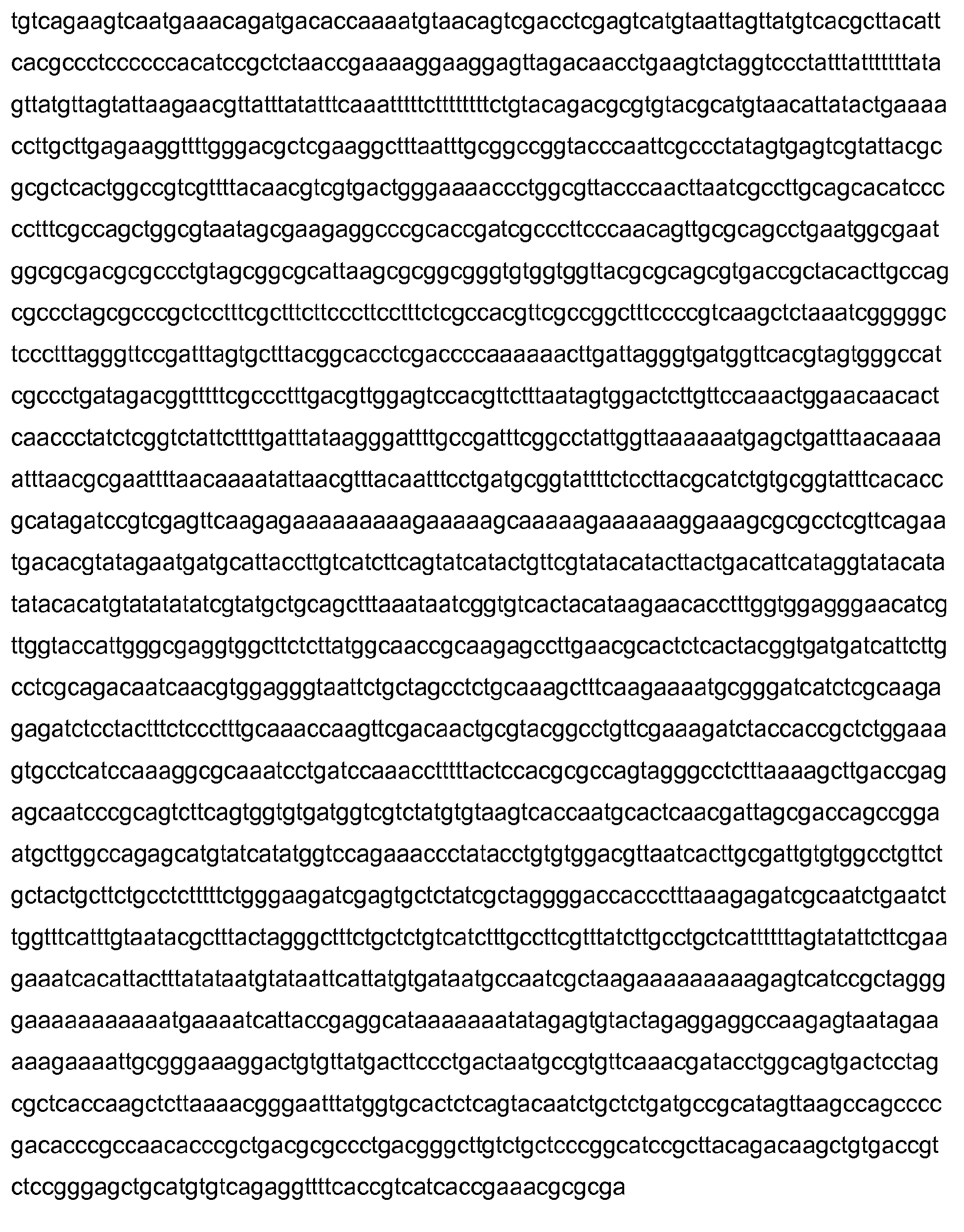

## Patentansprüche

1. Verfahren zur Identifizierung von Inhibitoren eines eukaryotischen Kaliumkanals, wobei
a) eine mutierte S. cerevisiae Zelle verwendet wird, die die drei endogenen Kaliumkanäle TRK1, TRK2 und TOK1 nicht exprimiert ;
b) in dieser mutierten S. cerevisiae Zelle ein eukaryotischer Kaliumkanal heterolog exprimiert wird;
c) die mutierte S. cerevisiae Zelle mit einer zu untersuchenden Substanz inkubiert wird;
und
d) der Effekt der zu untersuchenden Substanz auf den eukaryotischen Kaliumkanal bestimmt wird.

2. Mutierte S. cerevisiae Zelle, **dadurch gekennzeichnet, daß** die endogenen Kaliumkanäle TRK1, TRK2 und TOK1 nicht exprimiert werden.

3. Mutierte S. cerevisiae Zelle, **dadurch gekennzeichnet, daß** die Gene TRK1, TRK2 und TOK1 ausgeschaltet sind.

4. Mutierte S. cerevisiae Zelle hinterlegt unter DSM 13197.

5. Mutierte S. cerevisiae Zelle nach einem oder mehreren der Ansprüche 2-4, wobei die S. cerevisiae Zelle einen eukaryotischen Kaliumkanal heterolog exprimiert.

6. Mutierte S. cerevisiae Zelle nach einem oder mehreren der Ansprüche 2-5, wobei der eukaryotische Kaliumkanal ein humaner Kaliumkanal ist.

7. Mutierte S. cerevisiae Zelle nach einem oder mehreren der Ansprüche 2-6, wobei der eukaryotische Kaliumkanal ein HERG1, Kv1.5 oder gplRK1 ist.

8. Mutierte S. cerevisiae Zelle nach einem oder mehreren der Ansprüche 2-7, wobei der eukaryotische Kaliumkanal mutiert ist.

9. Verfahren zur Herstellung einer mutierten S. cerevisiae Zelle, die die Kaliumkanäle TRK, TRK2 und TOK1 nicht exprimiert, **dadurch gekennzeichnet, daß** die Gene TRK1, TRK2 und TOK1 durch knock out zerstört werden.

10. Verwendung einer mutierten S. cerevisiae Zelle nach einem oder mehreren der Ansprüche 2-8 zur Identifizierung von Substanzen, die die Aktivität des eukaryotischen Kaliumkanals inhibieren.

11. Verfahren zur Identifizierung von Aktivatoren eines eukaryotischen Kaliumkanals, wobei
a) eine mutierte S. cerevisiae Zelle verwendet wird, die die drei endogenen Kaliumkanäle TRK1, TRK2 und TOK1 nicht exprimiert;
b) in dieser mutierten S. cerevisiae Zelle ein eukaryotischer Kaliumkanal heterolog exprimiert wird;
c) die mutierte S. cerevisiae Zelle mit einer zu untersuchenden Substanz inkubiert wird;
und
d) der Effekt der zu untersuchenden Substanz auf den eukaryotischen Kaliumkanal bestimmt wird.

12. Verfahren zur Identifizierung von Aktivatoren eines eukaryotischen Kaliumkanals, wobei
a) eine mutierte S. cerevisiae Zelle verwendet wird, die die drei endogenen Kaliumkanäle TRK1, TRK2 und TOK1 nicht exprimiert;
b) in dieser mutierten S. cerevisiae Zelle ein eukaryotischer Kaliumkanal heterolog exprimiert wird;
c) die mutierten S. cerevisiae Zelle in Gegenwart eines Inhibitors des eukaryotischen Kaliumkanals mit einer zu untersuchenden Substanz inkubiert wird;
und
d) der Effekt der zu untersuchenden Substanz auf den eukaryotischen Kaliumkanal bestimmt wird.

13. Test-Kit enthaltend eine mutierte S. cerevisiae Zelle nach einem der Ansprüche 2-8.

14. Verfahren zur Herstellung eines Arzneimittels, wobei
a) ein Inhibitor eines eukaryotischen Kaliumkanals mit Hilfe eines Verfahrens nach Anspruch 1 identifiziert wird ,
b) der Inhibitor nach bekannten chemischen Verfahren hergestellt oder isoliert wird, und
c) der Inhibitor mit physiologisch verträglichen Zusatzstoffen versetzt wird.

15. Verfahren zur Herstellung eines Arzneimittels, wobei
a) ein Aktivator eines eukaryotischen Kaliumkanals mit Hilfe eines Verfahrens nach Anspruch 11 oder 12 identifiziert wird ,
b) der Aktivator nach bekannten chemischen Verfahren hergestellt oder isoliert wird, und
c) der Aktivator mit physiologisch verträglichen Zusatzstoffen versetzt wird.


SEQUENZPROTOKOLL

<110> Sanofi-Aventis Deutschland GmbH

<120> Kalium-Kanal-Mutanten der Hefe Saccharomyces cerevisiae
      und deren Verwendung für das Screening von
      eukaryotischen Kaliumkanälen

<130> AVE D-2000/A002

<140> PCT/EP01/00055
<141> 2001-01-05

<150> 10000651.5
<151> 2000-01-11

<160> 31

<170> PatentIn Ver. 2.1

<210> 1
<211> 3708
<212> DNA
<213> Saccharomyces cerevisiae

<400> 1
atgcatttta gaagaacgat gagtagagtg cccacattgg catctcttga aatacgatat 60
aaaaaatctt tcggccataa atttcgtgat tttattgctc tatgtggtca ctattttgct 120
ccagttaaaa aatatatctt ccccagtttt atcgcggttc actacttcta cacgatatcc 180
ctgacattaa taacttcaat cctgctatat cccattaaga ataccagata cattgataca 240
ttgtttttag cagcgggcgc agttacacaa ggtggcttaa atactgtgga tatcaacaat 300
ctaagcttat accaacaaat tgttctgtat atcgtatgct gcatatcaac accaattgca 360
gttcatagtt gcttggcatt tgtacggctt tactggtttg agcgctactt cgatggtatt 420
agagactctt ctagacgaaa ttttaagatg agaagaacga aaacaatctt agaaagggaa 480
ctaacagcaa gaaccatgac caagaataga acaggtaccc aaagaacgtc ttatcctagg 540
aaacaagcta aaacagatga tttccaagaa aaattgttca gcggagaaat ggttaataga 600
gatgagcagg actcagttca cagcgaccag aattctcatg acattagtag ggacagcagc 660
aataataata cgaatcacaa tggtagcagt ggcagtttag atgatttcgt taaggaagac 720
gaaacggatg acaatggaga atatcaggag aacaactcct actcgacggt aggtagttcg 780
tctaacacag ttgcagacga aagtttaaat cagaagccca agccaagcag tcttcggttt 840
gatgagccac acagcaaaca aagacccgca agagttccct cagagaaatt tgcaaaaaga 900
aggggttcaa gagatattag cccagccgat atgtatcgat ccattatgat gctacaaggt 960
aagcatgaag caactgctga agatgaaggt ccccctttag tcatcgggtc ccctgcggat 1020
ggcacaagat ataaaagtaa tgtcaataag ctaaagaagg ccaccggcat aaatggtaac 1080
aaaatcaaga ttcgagataa gggaaatgaa agtaacactg atcaaaattc cgtgtcaagt 1140
gaagcaaaca gtacggcgag cgtttcggac gaaagctcgt tacacacaaa ttttggtaac 1200
aaagtacctt cattaagaac aaatactcat agatcaaatt cgggcccgat agccattact 1260
gataacgcag aaacagacaa aaagcatggg ccatcaattc aattcgatat aactaaacct 1320
cctagaaaaa tttcaaaaag agtttcaacc ttcgatgatt tgaacccaaa atcttccgtt 1380
ctttatcgaa aaaaagcatc gaagaagtac ctcatgaaac attttcctaa agcgcggcga 1440
atacggcaac aaattaagag aaggctttct actggttcaa ttgagaaaaa cagcagtaac 1500
aatgtttcag atagaaaacc tattactgat atggatgatg atgatgatga cgatgacaac 1560
gacggcgata acaacgaaga atactttgct gacaacgaaa gcggcgatga agatgaacga 1620
gtacagcagt ctgaaccaca ttctgattca gaactcaaat cgcaccaaca acagcaagaa 1680
aaacaccaac tgcagcagaa cctgcaccgc atgtataaaa ccaaatcatt tgatgataat 1740
cgttcaagag cagttcctat ggaacgttcc aggaccatcg atatggcaga ggctaaggat 1800
ctaaatgagc tcgcaaggac gcctgatttt caaaaaatgg tctatcaaaa ttggaaagcc 1860
catcatagaa aaaaaccgaa ctttaggaag aggggatgga ataacaagat atttgaacat 1920
ggtccctatg catctgacag cgatcgcaat tatcctgata atagtaatac tggaaacagt 1980
attcttcatt acgcagagtc tattttacat catgatggct ctcataaaaa tggaagcgaa 2040
gaagcctctt ccgactctaa tgagaatatc tattccacga atggaggaag cgaccacaat 2100
ggtcttaaca actatcctac ttacaacgac gatgaagaag gctattatgg tttacatttc 2160
gataccgatt atgacctaga tcctcgtcat gatttatcta aaggcagtgg taaaacgtat 2220
ctatcatggc aaccaactat tggacgtaac tcaaacttcc ttggattaac aagagcccag 2280
aaagatgaat taggtggtgt cgagtacaga gcaatcaaac ttttatgcac catattggtt 2340
gtctactacg ttggatggca tattgttgct tttgttatgt tagtaccttg gattattttg 2400
aaaaagcatt atagtgaagt tgttagagat gatggtgttt cacctacatg gtggggattt 2460
tggacagcaa tgagtgcatt taatgattta ggtttgacat taactccaaa ttcaatgatg 2520
tcgtttaaca aagctgtata cccattgatc gttatgattt ggtttatcat tatcggaaat 2580

acagggtttc ccatccttct tagatgcatc atttggataa tgtttaaaat ttctcctgat 2640
ttatcacaga tgagagaaag tttaggtttt ctcttagacc atccacgtcg ttgtttcacc 2700
ttgctatttc ctaaggcagc tacatggtgg ctacttttaa cgcttgcagg attgaatata 2760
actgattgga ttttatttat tattctagat tttggctcaa cagttgtgaa atcattatcg 2820
aaaggctata gagtccttgt cggcctgttt caatctgtta gcacaagaac tgctggattc 2880
agcgttgtcg atttaagtca actgcatcct tctatccaag tctcctatat gctaatgatg 2940
tatgtctccg tattaccatt ggccatctct attcgacgga caaatgttta cgaggagcaa 3000
tctttaggac tatatggaga tatgggggga gaaccagaag atacggatac tgaagacgat 3060
ggtaacgatg aagatgacga cgaggaaaac gagagtcacg aaggtcaaag tagtcaaaga 3120
agtagttcga acaacaacaa caataacaac aggaaaaaga aaaagaaaaa gaaaactgaa 3180
aatccaaatg aaatatctac aaaatccttt atcggtgccc atttaaggaa acagctttca 3240
tttgacttgt ggtttctatt tttagggtta tttatcattt gcatttgtga aggggacaag 3300
ataaaggacg tacaagaacc aaactttaat atatttgcaa ttctttttga aattgttagc 3360
gcttacggta cagttgggct atcgctaggt tatccggaca ccaaccaatc gttttcaaga 3420
cagtttacta cattatctaa gttggtgatc atagctatgc tgatcagagg caagaataga 3480
ggtctaccat actcactgga tcgtgcaatt atcttgccta gtgatagact tgaacatatt 3540
gaccaccttg agggcatgaa attgaagaga caggctagaa ccaatacaga agacccaatg 3600
acggaacatt tcaagagaag tttcactgat gtgaaacatc gttggggagc tcttaagcgt 3660
aagaccacac attcccgaaa tcctaaaagg agcagcacaa cgctctaa 3708

<210> 2
<211> 2669
<212> DNA
<213> Saccharomyces cerevisiae

<400> 2
atgccaacag ctaagaggac gtcatccagg gcttcgttgg cactgccctt ccagttacgg 60
ttggtgcaca agaaatcatg gggccatcgg ctaagagact tcatttccgg gttcttaaaa 120
tcatgcagac ccattgctaa atacgttttc cccaacttca tcgtggtgca ctatatctac 180
ctgatcacgc tgtcgattat cgggtccatt ctgttatatc cgtgcaagaa cacggcgttc 240
atcgatgtgc tatttctggc tgctggagcg tctacacagg gcgggctggc caccaagagc 300
actaacgatt tcaacctgta ccagcagata gtggtgtacg tcattacatt gctgtccacg 360
cctatactta ttcatgggtt tttggccttt gtcaggctgt attggtttga aaggtacttc 420
gacaacatta gggatatctc caaacagaat tttaaactaa gaaggaccat gacgttgcaa 480
caaagggaac tatcgggcag cagtggcaat gccgctcgaa gtaggagttt caaggacaac 540
ctgttccgtg ggaagtttgt ttccagagaa gacccacgac aatccgcttc agatgtgccg 600
atggactctc ctgacacgtc cgcattgtcc tcaatctcac cgttgaatgt ttcctcctct 660
aaggaggaat ccagtgacac gcaaagctcg cctccaaact tctcaagtaa gcgccaaccc 720
tcagacgttg acccaagaga catttacaaa tcgataatga tgctacaaaa acaacaagag 780
aagagcaacg caaactccac ggattctttt tcgagcgaga ccaatggacc cgctttcatt 840
gtgcaggaac gtcatgagag aagagccccc cactgctcac tgaaacgcca ttctgtcctg 900
ccatcttctc aggaattgaa caagctagcc cagacgaaaa gtttccagaa attgcttggc 960
ttgcggagag atgaaggtga ccatgactac tttgacggtg ctcctcacaa atatatggtc 1020
accaagaaga aaaaaatatc tagaacgcaa tcatgtaaca tcccaacgta tactgcttca 1080
ccgagtccta aaacctcagg ccaagtagtt gaaaatcata gaaacttggc caagtcggcg 1140
ccttcatctt ttgttgatga ggagatgagc ttttcaccgc aagagtcttt gaatttacag 1200
ttccaagcgc acccgcccaa accaaaacga cgtgaaggtg atataggcca ccccttcacc 1260
agaacaatga gcaccaacta tctatcgtgg cagccaacct ttggcagaaa ctccgtcttc 1320
attggactca caaagcaaca aaaggaggaa ctcggcggtg tcgaatatcg tgctttgaga 1380
ttgctgtgct gcattctcat ggtatactac atcggattca acattttggc gtttgtgacc 1440
atcgttccat gggcctgtac gaggcaccac tactcagaga ttattagacg aaatggagtt 1500
tctccaacct ggtgggggtt tttcactgca atgagtgcat tcagcaactt gggtctgtct 1560
ttgaccgctg attcaatggt ttcctttgat actgcgccgt atccgctgat tttcatgatg 1620
ttcttcatca tcataggcaa tacaggcttc ccaattatgt tacgatttat catttggatc 1680
atgttcaaga cctcgagaga cctatctcag tttaaggaaa gtcttgggtt tctcttggat 1740
catccgcgca ggtgttttac gttgctgttc cccagcggcc ccacatggtg gctgtttaca 1800
actttagtcg tcttaaacgc tacggattgg attcttttca taattctgga tttcaactcc 1860
gctgtagtaa ggcaggttgc taaaggttat cgagctctca tgggcctctt ccagtctgta 1920
tgcacaagaa ctgctggatt caacgttgtt gacttaagta aattacaccc gtccattcag 1980
gtgtcttata tgctaatgat gtacgtttcg gtcctgccgc tggcgatttc cattagaaga 2040
acgaatgttt atgaggagca atcgttggga ctatacgata gtggacaaga tgacgaaaat 2100
atcacccacg aagacgatat aaaggaaaca gaccatgatg gcgaatccga agagcgagac 2160
actgtatcta caaagtccaa gccgaagaaa cagtccccaa aatcgtttgt tggtgctcat 2220
ttgaggaggc aactctcttt tgatttatgg tacctattcc ttggattatt tataatatgc 2280
atatgcgagg gcagaaaaat cgaagacgtt aataaacctg atttcaatgt ctttgctata 2340
ttgtttgaag ttgttagcgc ttatggtaca gtgggtttgt cattgggtta cccaaacacc 2400
aacacatcac tatctgccca gttcaccgta ttatcgaagc tagtcataat tgccatgcta 2460
ataagaggaa gaaatagagg tttaccatac actttggatc gtgccatcat gctgccaagt 2520

gacaaactgg aacaaattga tcgtttacaa gatatgaaag ctaagggtaa gttgttagcc 2580
aaagttggtg aggatccaat gactacttac gtcaaaaaga gatcccacaa actgaaaaaa 2640
atagcaacaa agttttgggg gaagcatta 2669

<210> 3
<211> 2076
<212> DNA
<213> Saccharomyces cerevisiae

<400> 3
atgacaaggt tcatgaacag ctttgccaaa caaacgctgg gatatggcaa tatggcgaca 60
gtggagcaag agagctcagc tcaggctgtt gattctcatt caaacaacac accgaagcaa 120
gctaagggtg ttcttgcaga ggaactaaag gatgcattgc ggttccggga cgaaagagtt 180
agtattatta atgcagagcc ttcttcaaca ctgttcgtct tttggtttgt ggtttcatgc 240
tatttccctg tgattactgc ctgcttgggt cccgtagcta acactatctc gatagcctgt 300
gtagttgaaa aatggagatc cttaaagaac aactccgtgg tgacaaatcc acgaagcaat 360
gacaccgatg ttttgatgaa tcaagtaaag acagtttttg atcctcctgg tatttttgcc 420
gttaatatca tctctttggt actgggtttt acgtcaaata ttatactaat gctacatttc 480
agtaagaagt tgacgtatct taaatctcag ttaataaata taacaggatg gacaatagct 540
ggagggatgc ttttggtgga cgtgattgta tgctccttga atgacatgcc cagcatctac 600
agtaagacta tcggattttg gtttgcctgt atcagttctg gtctatattt ggtatgcacc 660
attattttaa caatacattt tattggatat aaattaggaa aatatcctcc aacgttcaac 720
cttttgccca atgaaagaag tatcatggca tacactgtac tattgtcttt atggttgatt 780
tggggtgcgg gtatgtttag cggtttattg cacatcactt acggaaatgc attatatttc 840
tgcacggtat cattattaac cgtgggacta ggtgacatcc tgcccaagtc ggttggcgcc 900
aaaatcatgg ttttaatctt ttcgctatct ggtgttgtct tgatgggttt aatagtgttt 960
atgacaagat ccatcattca aaagtcctct ggcccaattt tctttttcca cagagttgaa 1020
aaaggcaggt ccaaatcgtg gaaacattat atggatagta gtaaaaattt atctgaaagg 1080
gaagcgttcg acttaatgaa gtgtatccga caaacggcct caaggaagca gcattggttt 1140
tctttgtcgg tgactattgc aattttcatg gctttttggt tattgggagc tcttgtattc 1200
aaattcgcag aaaattggtc gtacttcaat tgtatttact ttgtttcttt gtgcttatta 1260
accattggat acggagacta tgctccaagg actggtgcag gccgtgcttt ttttgtgatt 1320
tgggcgttgg gagccgtgcc attaatgggg gctatcctat ctacagtcgg tgatctgttg 1380
tttgacattt ccacttctct ggatattaag atcggtgaat cattcaataa taaagtcaag 1440
tccatcgttt ttaatgggcg tcaaagagca ctttccttta tggtgaacac tggagaaatt 1500
ttcgaagaat ctgacacagc tgatggtgat ctggaagaaa atacaacgag ctcacaatcc 1560
agtcaaattt ctgaattcaa cgataataat tcagaagaga atgattctgg agtgacatcc 1620
cctcctgcaa gcctgcaaga atcattttct tcattatcaa aagcatctag cccagaggga 1680
atacttcctc tagaatatgt ttcttctgct gaatatgcac tacaggactc ggggacctgt 1740
aatttaagga acttgcaaga gctacttaaa gccgtcaaaa aactacatcg gatatgtctg 1800
gcggataaag attacacact tagtttttcc gactggtcgt acattcataa actacatttg 1860
aggaacatta cagatattga ggagtacaca cgcggacccg aattttggat atcacctgat 1920
acgcccctca agttcccgtt aaatgaacct cattttgctt ttatgatgct tttcaagaac 1980
atagaagaat tagttggtaa tctagtagaa gacgaagagc tttataaagt tataagcaaa 2040
agaaaatttt tgggtgagca tagaaagaca ctttga 2076

<210> 4
<211> 3479
<212> DNA
<213> Homo sapiens

<400> 4
   atgccggtgc ggaggggcca cgtcgcgccg cagaacacct tcctggacac catcatccgc 60
   aagtttgagg gccagagccg taagttcatc atcgccaacg ctcgggtgga gaactgcgcc 120
   gtcatctact gcaacgacgg cttctgcgag ctgtgcggct actcgcgggc cgaggtgatg 180
   cagcgaccct gcacctgcga cttcctgcac gggccgcgca cgcagcgccg cgctgccgcg 240
   cagatcgcgc aggcactgct gggcgccgag gagcgcaaag tggaaatcgc cttctaccgg 300
   aaagatggga gctgcttcct atgtctggtg gatgtggtgc ccgtgaagaa cgaggatggg 360
   gctgtcatca tgttcatcct caatttcgag gtggtgatgg agaaggacat ggtggggtcc 420
   ccggctcatg acaccaacca ccggggcccc cccaccagct ggctggcccc aggccgcgcc 480
   aagaccttcc gcctgaagct gcccgcgctg ctggcgctga cggcccggga gtcgtcggtg 540
   cggtcgggcg gcgcgggcgg cgcgggcgcc ccgggggccg tggtggtgga cgtggacctg 600
   acgcccgcgg cacccagcag cgagtcgctg gccctggacg aagtgacagc catggacaac 660
   cacgtggcag ggctcgggcc cgcggaggag cggcgtgcgc tggtgggtcc cggctctccg 720
   ccccgcagcg cgcccggcca gctcccatcg ccccgggcgc acagcctcaa ccccgacgcc 780
   tcgggctcca gctgcagcct ggcccggacg cgctcccgag aaagctgcgc cagcgtgcgc 840
   cgcgcctcgt cggccgacga catcgaggcc atgcgcgccg gggtgctgcc cccgccaccg 900
   cgccacgcca gcaccggggc catgcaccca ctgcgcagcg gcttgctcaa ctccacctcg 960
   gactccgacc tcgtgcgcta ccgcaccatt agcaagattc cccaaatcac cctcaacttt 1020
   gtggacctca agggcgaccc cttcttggct tcgcccacca gtgaccgtga gatcatagca 1080
   cctaagataa aggagcgaac ccacaatgtc actgagaagg tcacccaggt cctgtccctg 1140
   ggcgccgacg tgctgcctga gtacaagctg caggcaccgc gcatccaccg ctggaccatc 1200
   ctgcattaca gccccttcaa ggccgtgtgg gactggctca tcctgctgct ggtcatctac 1260
   acggctgtct tcacacccta ctcggctgcc ttcctgctga aggagacgga agaaggcccg 1320
   cctgctaccg agtgtggcta cgcctgccag ccgctggctg tggtggacct catcgtggac 1380
   atcatgttca ttgtggacat cctcatcaac ttccgcacca cctacgtcaa tgccaacgag 1440
   gaggtggtca gccaccccgg ccgcatcgcc gtccactact tcaagggctg gttcctcatc 1500
   gacatggtgg ccgccatccc cttcgacctg ctcatcttcg gctctggctc tgaggagctg 1560
   atcgggctgc tgaagactgc gcggctgctg cggctggtgc gcgtggcgcg gaagctggat 1620
   cgctactcag agtacggcgc ggccgtgctg ttcttgctca tgtgcacctt tgcgctcatc 1680
   gcgcactggc tagcctgcat ctggtacgcc atcggcaaca tggagcagcc acacatggac 1740
   tcacgcatcg gctggctgca caacctgggc gaccagatag gcaaacccta caacagcagc 1800
   ggcctgggcg gcccctccat caaggacaag tatgtgacgg cgctctactt caccttcagc 1860
   agcctcacca gtgtgggctt cggcaacgtc tctcccaaca ccaactcaga gaagatcttc 1920
   tccatctgcg tcatgctcat tggctccctc atgtatgcta gcatcttcgg caacgtgtcg 1980
   gccatcatcc agcggctgta ctcgggcaca gcccgctacc acacacagat gctgcgggtg 2040
   cgggagttca tccgcttcca ccagatcccc aatcccctgc gccagcgcct cgaggagtac 2100
   ttccagcacg cctggtccta caccaacggc atcgacatga acgcggtgct gaagggcttc 2160
   cctgagtgcc tgcaggctga catctgcctg cacctgaacc gctcactgct gcagcactgc 2220
   aaacccttcc gaggggccac caagggctgc cttcgggccc tggccatgaa gttcaagacc 2280
   acacatgcac cgccagggga cacactggtg catgctgggg acctgctcac cgccctgtac 2340
   ttcatctccc ggggctccat cgagatcctg cggggcgacg tcgtcgtggc catcctgggg 2400
   aagaatgaca tctttgggga gcctctgaac ctgtatgcaa ggcctggcaa gtcgaacggg 2460
   gatgtgcggg ccctcaccta ctgtgaccta cacaagatcc atcggacgga cctgctggag 2520
   gtgctggaca tgtaccctga gttctccgac cacttctggt ccagcctgga gatcaccttc 2580
   aacctgcgag ataccaacat gatcccgggc tcccccggca gtacggagtt agagggtggc 2640
   ttcagtcggc aacgcaagcg caagttgtcc ttccgcaggc gcacggacaa ggacacggag 2700
   cagccagggg aggtgtcggc cttggggccg ggccgggcgg gggcagggcc gagtagccgg 2760
   ggccggccgg gggggccgtg gggggagagc ccgtccagtg gcccctccag ccctgagagc 2820
   agtgaggatg agggcccagg ccgcagctcc agccccctcc gcctggtgcc cttctccagc 2880
   cccaggcccc ccggagagcc gccgggtggg gagcccctga tggaggactg cgagaagagc 2940
   agcgacactt gcaaccccct gtcaggcgcc ttctcaggag tgtccaacat tttcagcttc 3000
   tggggggaca gtcggggccg ccagtaccag gagctccctc gatgccccgc ccccaccccc 3060
   agcctcctca acatccccct ctccagcccg ggtcggcggc ccgggggcga cgtggagagc 3120
   aggctggatg ccctccagcg ccagctcaac aggctggaga cccggctgag tgcagacatg 3180
   gccactgtcc tgcagctgct acagaggcag atgacgctgg tcccgcccgc ctacagtgct 3240
   gtgaccaccc cggggcctgg ccccacttcc acatccccgc tgttgcccgt cagccccctc 3300
   cccaccctca ccttggactc gctttctcag gtttcccagt tcatggcgtg tgaggagctg 3360
   cccccggggg ccccagagct tccccaagaa ggccccacac gacgcctctc cctaccgggc 3420
   cagctggggg ccctcacctc ccagcccctg cacagacacg gctcggaccc gggcagtta 3479

<210> 5
<211> 1836
<212> DNA
<213> Homo sapiens

<400> 5
atggagatcg ccctggtgcc cctggagaac ggcggtgcca tgaccgtcag aggaggcgat 60
gaggcccggg caggctgcgg ccaggccaca gggggagagc tccagtgtcc cccgacggct 120
gggctcagcg atgggcccaa ggagccggcg ccaaaggggc gcgcgcagag agacgcggac 180
tcgggagtgc ggcccttgcc tccgctgccg gacccgggag tgcggccctt gcctccgctg 240
ccagaggagc tgccacggcc tcgacggccg cctcccgagg acgaggagga agaaggcgat 300
cccggcctgg gcacggtgga ggaccaggct ctgggcacgg cgtccctgca ccaccagcgc 360
gtccacatca acatctccgg gctgcgcttt gagacgcagc tgggcaccct ggcgcagttc 420
cccaacacac tcctggggga ccccgccaag cgcctgccgt acttcgaccc cctgaggaac 480
gagtacttct tcgaccgcaa ccggcccagc ttcgacggta tcctctacta ctaccagtcc 540
gggggccgcc tgcgaggggt caacgtctcc ctggacgtgt tcgcggacga gatacgcttc 600
taccagctgg gggacgaggc catggagcgc ttccgcgagg atgagggctt cattaaagaa 660
gaggagaagc ccctgccccg caacgagttc cagcgccagg tgtggcttat cttcgagtat 720
ccggagagct ctgggtccgc gcgggccatc gccatcgtct cggtcttggt tatcctcatc 780
tccatcatca ccttctgctt ggagaccctg cctgagttca gggatgaacg tgagctgctc 840
cgccaccctc cggcgcccca ccagcctccc gcgcccgccc ctggggccaa cggcagcggg 900
gtcatggccc ccgcctctgg ccctacggtg gcaccgctcc tgcccaggac cctggccgac 960
cccttcttca tcgtggagac cacgtgcgtg atctggttca ccttcgagct gctcgtgcgc 1020
ttcttcgcct gccccagcaa ggcagggttc tcccggaaca tcatgaacat catcgatgtg 1080

gtggccatct tcccctactt catcaccctg ggcaccgaac tggcagagca gcagccaggg 1140
ggcggaggag gcggccagaa tgggcagcag gccatgtccc tggccatcct ccgagtcatc 1200
cgcctggtcc gggtgttccg catcttcaag ctctcccgcc actccaaggg gctgcagatc 1260
ctgggcaaga ccttgcaggc ctccatgagg gagctggggc tgctcatctt cttcctcttc 1320
atcggggtca tcctcttctc cagtgccgtc tacttcgcag aggctgacaa ccagggaacc 1380
catttctcta gcatccctga cgccttctgg tgggcagtgg tcaccatgac cactgtgggc 1440
tacggggaca tgaggcccat cactgttggg ggcaagatcg tgggctcgct gtgtgccatc 1500
gccggggtcc tcaccattgc cctgcctgtg cccgtcatcg tctccaactt caactacttc 1560
taccaccggg aaacggatca cgaggagccg gcagtcctta aggaagagca gggcactcag 1620
agccaggggc cggggctgga cagaggagtc cagcggaagg tcagcgggag caggggatcc 1680
ttctgcaagg ctggggggac cctggagaat gcagacagtg cccgaagggg cagctgcccc 1740
ctagagaagt gtaacgtcaa ggccaagagc aacgtggact tgcggaggtc cctttatgcc 1800
ctctgcctgg acaccagccg ggaaacagat ttgtga 1836


<210> 6
<211> 1284
<212> DNA
<213> Homo sapiens

<400> 6
   atgggcagtg tgcgaaccaa ccgctatagc attgtctctt cggaagagga cggcatgaag 60
   ttggccacca tggcagttgc caatggcttt gggaatggga aaagtaaagt ccacactcgg 120
   caacagtgta ggagccgctt tgtgaagaaa gatggccact gtaatgttca gttcatcaac 180
   gttggggaaa agggacaacg gtaccttgct gacattttta ctacgtgtgt ggacattcgc 240
   tggcggtgga tgctggttat cttttgccta gcttttgttc tctcgtggct gttttttggc 300
   tgtgtgtttt ggctgatagc tttgctccat ggagatctgg atgcatctaa ggagagcaaa 360
   gcctgtgtgt ctgaggtcaa cagcttcaca gctgcctttc ttttctccat tgagacccag 420
   acaaccatcg gctatgggtt ccgatgtgtc acggatgaat gcccgattgc ggtgttcatg 480
   gttgtgttcc agtcaattgt gggctgcatt attgatgctt ttatcattgg tgccgtcatg 540
   gcaaagatgg caaagccaaa gaaaagaaat gagactcttg tcttcagtca caatgctgtg 600
   attgccatga gagatggcaa gctgtgtttg atgtggcgag taggcaacct tcggaaaagc 660
   cacttggtag aagctcatgt tcgagcccag ctcctcaaat ccagaattac ttctgaaggg 720
   gaatacatcc ccttggatca aatagacatc aatgttggct ttgacagtgg aattgaccgt 780
   atatttctgg tatccccaat cactattgtc catgaaatag atgaagatag tcctttatat 840
   gatttgagca agcaggacat tgataatgca gactttgaaa ttgttgtgat actagaaggc 900
   atggtggaag ccactgccat gacaacacag tgtcgtagtt cttatttggc caacgagatc 960
   ctttggggcc accgctatga gccagtgctc tttgaggaga agcactacta taaagtggac 1020
   tattcgaggt ttcataagac ttacgaagta cccaacactc ccctttgtag tgccagagac 1080
   ttagcagaaa agaaatatat tctctcaaat gctaactcat tttgctatga aaatgaagtt 1140
   gcccttacaa gcaaagagga agatgacagt gaaaatgggg ttccagaaag caccagtaca 1200
   gacacacctc ctgacatcga ccttcacaac caggcaagtg tacctctaga gcccagaccc 1260
   ttacggcgag aatcggagat atga 1284


<210> 7
<211> 34
<212> DNA
<213> Künstliche Sequenz

<220>
<223> Beschreibung der künstlichen Sequenz:misc-feature

<220>
<221> misc_feature
<222> (1)..(34)
<223> Oligonucleotide

<400> 7
gcggatccat gcattttaga agaacgatga gtag 34


<210> 8
<211> 21
<212> DNA
<213> Künstliche Sequenz

<220>
<223> Beschreibung der künstlichen Sequenz:misc-feature

<220>
<221> misc_feature
<222> (1)..(21)
<223> Oligonukleotide

<400> 8
aggttctgct gcagttggtg t                                                21


<210> 9
<211> 21
<212> DNA
<213> Künstliche Sequenz

<220>
<223> Beschreibung der künstlichen Sequenz:misc-feature

<220>
<221> misc_feature
<222> (1)..(21)
<223> Oligonukleotide

<400> 9
acaccaactg cagcagaacc t                                                21


<210> 10
<211> 29
<212> DNA
<213> Künstliche Sequenz

<220>
<223> Beschreibung der künstlichen Sequenz:misc-feature

<220>
<221> misc_feature
<222> (1)..(29)
<223> Oligonukleotide

<400> 10
cgctcgagtt agagcgttgt gctgctcct                                        29


<210> 11
<211> 22
<212> DNA
<213> Künstliche Sequenz

<220>
<223> Beschreibung der künstlichen Sequenz:misc-feature

<220>
<221> misc_feature
<222> (1)..(22)
<223> Oligonukleotide

<400> 11
ccttaccatt agcatcactg at                                               22


<210> 12
<211> 21
<212> DNA
<213> Künstliche Sequenz

<220>
<223> Beschreibung der künstlichen Sequenz:misc-feature

<220>
<221> misc_feature
<222> (1)..(21)
<223> Oligonukleotide

<400> 12
ctattaacca tttctccgct g                                                21


<210> 13
<211> 24
<212> DNA
<213> Künstliche Sequenz

<220>
<223> Beschreibung der künstlichen Sequenz:misc-feature

<220>
<221> misc_feature
<222> (1)..(24)
<223> Oligonukleotide

<400> 13
gatttatctt cgtttcctgc aggt                                             24


<210> 14
<211> 31
<212> DNA
<213> Künstliche Sequenz

<220>
<223> Beschreibung der künstlichen Sequenz:misc-feature

<220>
<221> misc_feature
<222> (1)..(31)
<223> Oligonukleotide

<400> 14
cacgtacgtc cagcacaatt tcacaacagc t                                     31


<210> 15
<211> 30
<212> DNA
<213> Künstliche Sequenz

<220>
<223> Beschreibung der künstlichen Sequenz:misc-feature

<220>
<221> misc_feature
<222> (1)..(30)
<223> Oligonukleotide

<400> 15
cagtcgacct ggatgacgtc ctcttagctg                                       30


<210> 16
<211> 29
<212> DNA
<213> Künstliche Sequenz

<220>
<223> Beschreibung der künstlichen Sequenz:misc-feature

<220>

<221> misc_feature
<222> (1)..(29)
<223> Oligonukleotide

<400> 16
cagatatcat gctgccaagt gacaaactg                                        29


<210> 17
<211> 28
<212> DNA
<213> Künstliche Sequenz

<220>
<223> Beschreibung der künstlichen Sequenz:misc-feature

<220>
<221> misc_feature
<222> (1)..(28)
<223> Oligonukleotide

<400> 17
tcactagttg ttgatggctt tggttggt                                         28


<210> 18
<211> 22
<212> DNA
<213> Künstliche Sequenz

<220>
<223> Beschreibung der künstlichen Sequenz:misc-feature

<220>
<221> misc_feature
<222> (1)..(22)
<223> Oligonukleotide

<400> 18
gcgaagaata ggatgagatg tg                                               22


<210> 19
<211> 20
<212> DNA
<213> Künstliche Sequenz

<220>
<223> Beschreibung der künstlichen Sequenz:misc-feature

<220>
<221> misc_feature
<222> (1)..(20)
<223> Oligonukleotide

<400> 19
ttgtcgtggg tcttctctgg                                                  20


<210> 20
<211> 23
<212> DNA
<213> Künstliche Sequenz

<220>
<223> Beschreibung der künstlichen Sequenz:misc-feature

<220>
<221> misc_feature

<222> (1)..(23)
<223> Oligonukleotide

<400> 20
gctacctttg ccatgtttca gaa                                              23


<210> 21
<211> 31
<212> DNA
<213> Künstliche Sequenz

<220>
<223> Beschreibung der künstlichen Sequenz:misc-feature

<220>
<221> misc_feature
<222> (1)..(31)
<223> Oligonukleotide

<400> 21
cacgtacggc aaatttatcg agactctgcg a                                     31


<210> 22
<211> 29
<212> DNA
<213> Künstliche Sequenz

<220>
<223> Beschreibung der künstlichen Sequenz:misc-feature

<220>
<221> misc_feature
<222> (1)..(29)
<223> Oligonukleotide

<400> 22
aggtcgacca tattgccata tcccagcgt                                        29


<210> 23
<211> 21
<212> DNA
<213> Künstliche Sequenz

<220>
<223> Beschreibung der künstlichen Sequenz:misc-feature

<220>
<221> misc_feature
<222> (1)..(21)
<223> Oligonukleotide

<400> 23
tggatatcac ctgatacgcc c                                                21


<210> 24
<211> 35
<212> DNA
<213> Künstliche Sequenz

<220>
<223> Beschreibung der künstlichen Sequenz:misc-feature

<220>
<221> misc_feature
<222> (1)..(35)

<223> Oligonukleotide

<400> 24
caactagtgc ataccagtag tatgagacat gcttg                                35

<210> 25
<211> 23
<212> DNA
<213> Künstliche Sequenz

<220>
<223> Beschreibung der künstlichen Sequenz:misc-feature

<220>
<221> misc_feature
<222> (1)..(23)
<223> Oligonukleotide

<400> 25
cctgagtact cagtaccatc ttg                                             23

<210> 26
<211> 18
<212> DNA
<213> Künstliche Sequenz

<220>
<223> Beschreibung der künstlichen Sequenz:misc-feature

<220>
<221> misc_feature
<222> (1)..(18)
<223> Oligonukleotide

<400> 26
ctgtagatgc tgggcatg                                                   18

<210> 27
<211> 27
<212> DNA
<213> Künstliche Sequenz

<220>
<223> Beschreibung der künstlichen Sequenz:misc-feature

<220>
<221> misc_feature
<222> (1)..(27)
<223> Oligonukleotide

<400> 27
tacgtcgaca tggagatcgc cctggtg                                         27

<210> 28
<211> 28
<212> DNA
<213> Künstliche Sequenz

<220>
<223> Beschreibung der künstlichen Sequenz:misc-feature

<220>
<221> misc_feature
<222> (1)..(28)
<223> Oligonukleotide

<400> 28
tacgtcgaca tctgtttccc ggctggtg                                          28


<210> 29
<211> 25
<212> DNA
<213> Künstliche Sequenz

<220>
<223> Beschreibung der künstlichen Sequenz:misc-feature

<220>
<221> misc_feature
<222> (1)..(25)
<223> Oligonukleotide

<400> 29
tacatcgata tgccggtgcg gaggg                                             25


<210> 30
<211> 24
<212> DNA
<213> Künstliche Sequenz

<220>
<223> Beschreibung der künstlichen Sequenz:misc-feature

<220>
<221> misc_feature
<222> (1)..(24)
<223> Oligonukleotide

<400> 30
tacgtcgaca ctgcccgggt ccga                                              24


<210> 31
<211> 1119
<212> DNA
<213> Homo sapiens

<400> 31
atgttcaaac atcttcggaa atgggtcgtc actcgctttt ttgggcattc tcggcaaaga 60
gcaaggctag tctccaaaga tggaaggtgc aacatagaat ttggcaatgt ggaggcacag 120
tcaaggttta tattctttgt ggacatctgg acaacggtac ttgacctcaa gtggagatac 180
aaaatgacca ttttcatcac agccttcttg gggagttggt ttttctttgg tctcctgtgg 240
tatgcagtag cgtacattca caaagacctc ccggaattcc atccttctgc caatcacact 300
ccctgtgtgg agaatattaa tggcttgacc tcagcttttc tgttttctct ggagactcaa 360
gtgaccattg gatatggatt caggtgtgtg acagaacagt gtgccactgc catttttctg 420
cttatctttc agtctatact tggagttata atcaattctt tcatgtgtgg ggccatctta 480
gccaagatct ccaggcccaa aaaacgtgcc aagaccatta cgttcagcaa gaacgcagtg 540
atcagcaaac ggggagggaa gctttgcctc ctaatccgag tggctaatct caggaagagc 600
cttcttattg gcagtcacat ttatggaaag cttctgaaga ccacagtcac tcctgaagga 660
gagaccatta ttttggacca gatcaatatc aactttgtag ttgacgctgg gaatgaaaat 720
ttattcttca tctccccatt gacaatttac catgtcattg atcacaacag ccctttcttc 780
cacatggcag cggagaccct tctccagcag gactttgaat tagtggtgtt tttagatggc 840
acagtggagt ccaccagtgc tacctgccaa gtccggacat cctatgtccc agaggaggtg 900
ctttggggct accgttttgc tcccatagta tccaagacaa aggaagggaa ataccgagtg 960
gatttccata actttagcaa gacagtggaa gtggagaccc ctcactgtgc catgtgcctt 1020
tataatgaga aagatgttag agccaggatg aagagaggct atgacaaccc caacttcatc 1080
ttgtcagaag tcaatgaaac agatgacacc aaaatgtaa                              1119